(19) Europäisches Patentamt
European Patent Office
Office européen des brevets

(11) Veröffentlichungsnummer: **0 307 553 B1**

(12) # EUROPÄISCHE PATENTSCHRIFT

(45) Veröffentlichungstag der Patentschrift: **15.06.94**

(51) Int. Cl.5: **C07K 7/00**, A61K 37/02

(21) Anmeldenummer: **88109774.5**

(22) Anmeldetag: **20.06.88**

Die Akte enthält technische Angaben, die nach dem Eingang der Anmeldung eingereicht wurden und die nicht in dieser Patentschrift enthalten sind.

(54) **Modifizierte Splenopentine, Verfahren zu ihrer Herstellung und ihre Verwendung.**

(30) Priorität: **19.06.87 DD 303975**
**23.12.87 DD 311080**
**08.01.88 DD 312144**

(43) Veröffentlichungstag der Anmeldung:
**22.03.89 Patentblatt 89/12**

(45) Bekanntmachung des Hinweises auf die Patenterteilung:
**15.06.94 Patentblatt 94/24**

(84) Benannte Vertragsstaaten:
**AT BE CH DE ES FR GB IT LI NL SE**

(56) Entgegenhaltungen:
**EP-A- 0 025 897** **EP-A- 0 037 246**
**EP-A- 0 067 425** **EP-A- 0 135 722**
**EP-A- 0 144 103** **EP-A- 0 166 612**

**CHEMICAL ABSTRACTS, Band 105, 1986, Seite 574, Zusammenfassung Nr. 189225c, Columbus, Ohio, US; T. ABIKO et al.: "Syntheses and effects of [Glu34]-thymopoietin II fragment 32-45 and its analogs on the impaired T-cell transformation in a patient with common variable immunodeficiency"**

(73) Patentinhaber: **BERLIN-CHEMIE AG**
**Glienicker Weg 125-127**
**D-12489 Berlin(DE)**

(72) Erfinder: **Forner, Klaus, Dr.rer.nat.**
**Florastr. 34**
**DD-1100 Berlin(DD)**
Erfinder: **Ehrlich, Angelika**
**Pekrunstr. 69**
**DD-1140 Berlin(DD)**
Erfinder: **Diezel, Wolfgang, Prof.Dr.sc.med.**
**Anton-Saefkow-Platz 3**
**DD-1156 Berlin(DD)**
Erfinder: **Eckert, Rolf, Dr.rer.nat.**
**Anklamer Str. 60**
**DD-1040 Berlin(DD)**
Erfinder: **Euthin, Elke, Dr. rer.nat.**
**Plönzeile 10**
**DD-1166 Berlin(DD)**
Erfinder: **Krause, Eberhard, Dr. rer.nat.**
**Robert-Uhrig-Str. 18**
**DD-1136 Berlin(DD)**
Erfinder: **Slonina, Peter**
**Rudolf-Seiffert-Str. 17**
**DD-1156 Berlin(DD)**
Erfinder: **Volk, Hans-Dieter, Dr. med.**
**Heinrich-Rau-Str. 44**
**DD-1140 Berlin(DD)**

EP 0 307 553 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Repke, Heinrich, Dr. sc.nat.**
**Ludwig-Renn-Str. 48**
**DD-1142 Berlin(DD)**
Erfinder: **Georgi, Monika**
**Frankfurter Allee 178**
**DD-1130 Berlin(DD)**
Erfinder: **Leidert, Martina**
**Amanlifweg 10**
**DD-1140 Berlin(DD)**
Erfinder: **Bienert, Michael, Dr. sc.nat.**
**Parkstr. 14**
**DD-1114 Berlin(DD)**
Erfinder: **Ovcinnikov, Michail**
**Ul. Ostrovitenova 16, Korp. 3., Whg. 26**
**Moskau(SU)**
Erfinder: **Schmidt, Ralph**
**Crusemarkstr. 33**
**DD-1100 Berlin(DD)**
Erfinder: **Schütt, Manfred, Dr. rer.nat.**
**Karower Chaussee 15**
**DD-1115 Berlin(DD)**
Erfinder: **Mentel, Renate, Dr. rer.nat.**
**Breitscheidstr. 14**
**DD-2200 Greifswald(DD)**
Erfinder: **Breustedt, Winfried, Dr.med.**
**Vetschauer Allee 4**
**DD-1187 Berlin(DD)**

(74) Vertreter: **Patentanwälte Beetz - Timpe - Siegfried Schmitt-Fumian - Mayr**
**Steinsdorfstrasse 10**
**D-80538 München (DE)**

## Beschreibung

Die Erfindung betrifft neue Derivate des Pentapeptids Splenopentin Arg-Lys-Glu-Val-Tyr, Verfahren zu ihrer Herstellung und Reinigung sowie deren Verwendung als immunbiologisch wirksame Arzneimittel.
Splenopentin und seine Derivate werden als Immunregulatoren in der medizinischen Therapie eingesetzt.
Das Anwendungsgebiet der Erfindung ist die pharmazeutische Industrie.
Im Ergebnis umfangreicher Untersuchungen zur Isolierung und Charakterisierung von Peptiden, die das Immunsystem beeinflussen, wurden in den vergangenen Jahren u. a. die Thymopoietine und das Splenin (T. Audhya et al., Biochemistry 20 (1981) 6195) aus den Thymus bzw. der Milz isoliert.
Bei unterschiedlicher Organherkunft weisen diese Peptide eine weitgehende Strukturähnlichkeit auf; sie bestehen aus 49 Aminosäuren und unterscheiden sich in ihrer Sequenz nur in Position 34 durch Asparaginsäure bzw. Glutaminsäure voneinander. Hinsichtlich ihrer biologischen Aktivität unterscheiden sie sich dagegen deutlich. So beeinflussen die Thymopoietine die neuromuskuläre Transmission (M. P. Scheid et al., J. Exp. Med. 147, (1978) 1727), stimulieren die Differenzierung früher T-Zellen und inhibieren die Differenzierung früher B-Zellen.

Splenin stimuliert dagegen die Differenzierung von T- und B-Zellen und weist keine Neuroaktivität auf. Die biologische Wirkqualität des Gesamtmoleküls Splenin wird durch seine Teilsequenz 32-36 Arg-Lys-Glu-Val-Tyr (Splenopentin) reproduziert.
Die Herstellung von biologisch aktiven Substanzen setzt in immer stärkerem Maße die gleichzeitige Entwicklung hochempfindlicher und wirtschaftlich effektiver chromatographischer Reinigungsmethoden voraus. Vorrangig dabei ist der Einsatz solcher Verfahren, die einen hohen Substanzdurchsatz, eine gleichmäßig gute Wiederfindungsrate und hohe Systemauflösung bei Verwendung einfacher Träger- und Laufmittelsysteme aufweisen.
Die chromatographische Trennung unterschiedlich acylierter Splenopentin-Peptide erfordert auf Grund der geringen Ladungs- und damit Polaritätsunterschiede einer Vielzahl von Komponenten die Anwendung aufwendiger und kostspieliger Chromatographietechniken. Vorzugsweise kommen dabei HPLC-Verfahren auf der Basis von RP-Kieselgelen sowie Methoden der polaren Adsorptions- oder Verteilungschromatographie an Normalkieselgelphasen zum Einsatz.
Nach DE-A1-3 421 614 erfolgt die chromatographische Reinigung von Splenopentinen an Kieselgel-Normalphssen in methylenchloridhaltigen Laufmitteln. Es wurde weiterhin bereits vorgeschlagen, die chromatographische Trennung des synthetischen Splenopentin-Rohproduktes durch Adsorptions- bzw. Verteilungschromatographie an Kieselgel 60 unter Einsatz von pyridinhaltigen organischen Laufmitteln durchzuführen. Ähnliche Trennverfahren sind in DE-A1-2 804 566 zur Reinigung von Thymopentin bzw. Monoacetylthymopentin angegeben.
Nachteilig an Verfahren auf der Basis von RP- oder Normalphasen-Kieselgel ist der hohe apparative oder materialtechnische Aufwand, der ihren Einsatz bei der Herstellung großer Mengen hochreiner Produkte ausschließt. Weiterhin sind häufig die geringe Kapazität des Systems, niedrige Wiederfindungsraten durch unspezifische Adsorption sowie unzureichende Auflösung limitierende Faktoren für ihre Anwendung.

Übliche ionenaustauschchromatographische Reinigungsverfahren für Peptide sind durch den Einsatz von Salzen während des Elutionsprozesses gekennzeichnet. So erfolgt die in DE-A1-2 732 587 sowie in DE-A1-2 617 646 beschriebene chromatographische Reinigung von Nona- bzw. Decapeptiden durch Einsatz von Carboxymethylcellulose mit Natriumchlorid bzw. Ammoniumacetat enthaltenden Elutionsmitteln. Zur Reinigung der Substanzen ist entsprechend nachfolgend immer eine aufwendige Entsalzung notwendig.

EP-A-25897 betrifft dem Splenopentin analoge Pentapeptide, wobei in den dortigen Beispielen 25, 26, 30 und 32-36 die Verbindungen Arg-Lys-Glu-Val-Tyr-OMe, Arg-Lys-Glu-Val-Tyr-OH, Arg-Lys-Glu-Val-Tyr-O-cyclohexyl, Arg-Lys-Glu-Val-Tyr-O-n-hexyl, Arg-Lys-Glu-Val-Tyr-O-n-butyl, Arg-Lys-Glu-Val-Tyr-O-$CH(CH_3)_2$, Arg-Lys-Glu-Val-Tyr-NH-$C_2H_5$ bzw. Arg-Lys-Glu-Val-Tyr-NH-$CH_2$-$CH(CH_3)_2$ genannt sind. Für diese Verbindungen ist pauschal angegeben, daß sie sich zur Beeinflussung der Reifung von T-Lymphocyten eignen. Diese Wirksamkeit ist allerdings nicht durch experimentelle Ergebnisse nachgewiesen. Gleiches gilt für die angegebene erhöhte Lebensdauer in Leberhomogenisaten gegenüber den natürlichen Thymuspeptiden. Auf der anderen Seite war bekannt (Int. J. Peptide Protein Res. **14** (1979) 479 und Peptides 1988, Proceedings of the 20th Europ. Pept. Symp., Walter de Gruyter Verlag, Berlin, N.Y. 1989, 745), daß Arg-Lys-Peptide extrem schnell durch Aminopeptidasen zu biologisch inaktiven Peptidfragmenten abgebaut werden, worauf eine C-terminale Modifizierung kaum Einfluß haben sollte.

Der Erfindung liegt die Aufgabe zugrunde, neue immunologisch wirksame Derivate des Splenopentins (SP5), Verfahren zu ihrer Herstellung und Reinigung sowie immunbiologisch wirksame Arzneimittel zur Verfügung zu stellen.

Die Aufgabe wird anspruchsgemäß gelöst. Vorteilhafte Weiterbildungen sind Gegenstand der abhängigen Ansprüche.

Die erfindungsgemäßen neuen modifizierten Splenopentine besitzen die Formel I,

$R^1$-Arg-Lys($R^2$)-Glu-Val-Tyr($R^3$)$R^4$ (I),

worin bedeuten:

$R^1$: Wasserstoff, $C_{1-7}$-Alkyl, $C_{5-12}$-Aryl, $C_{6-20}$-Alkaryl, $C_{6-20}$-Aralkyl, $C_{1-18}$-Alkanoyl, $C_{1-7}$-Alkenyl, $C_{1-7}$-Alkinyl, $C_{6-12}$-Polyhydroxyalkanoyl, -CO($CH_2$)$_n$-COOH (n = 1-8), -CO(CHOH)$_n$-COOH (n = 1-12), -CO-($CH_2$)$_n$-CO-Arg-Lys-Glu-Val-Tyr (n = 1-8) oder -CO-(CHOH)$_n$-CO-Arg-Lys-Glu-Val-Tyr (n = 1-12);

$R^2$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl, $C_{1-7}$-Alkinyl, $C_{6-12}$-Polyhydroxyalkanoyl, -CO($CH_2$)$_n$-COOH (n = 1-8), -CO(CHOH)$_n$-COOH (n = 1-12), -CO-($CH_2$)$_n$-CO-($N^\epsilon$-Lys)-SP5 (n = 1-8) oder -CO(CHOH)$_n$-CO-($N^\epsilon$-Lys) = SP5 (n = 1-12);

$R^3$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl oder $C_{1-7}$-Alkinyl

und

$R^4$: OH, $NH_2$, $NHR^5$, $NR^5_2$ oder $OR^5$, worin $R^5$ bedeutet: $C_{1-18}$-Alkyl, $C_{1-7}$-Alkenyl, $C_{1-7}$-Alkinyl, $C_{6-20}$-Aralkyl, $C_{6-20}$-Alkaryl, -(CHOH)$_n$-Polyhydroxyalkyl (n = 1-8), -($CH_2$)$_m$-NH-Tyr-Val-Glu-Lys-Arg (m = 1-18) -($CH_2CH_2$-O)$_m$-Tyr-Val-Glu-Lys-Arg (m = 1-10000) oder -($CH_2CH_2$-O)$_m$-NH-Tyr-Val-Glu-Lys-Arg (m = 1-10000), wobei Splenopentin ($R^1$ = $R^2$ = $R^3$ = H; $R^4$ = OH)

ausgenommen ist und $N^\alpha$-Arg-Substitution, $N^\epsilon$-Lys-Substitution oder $N^\alpha$-Arg- und $N^\epsilon$-Lys-Substitution vorliegt.

Erfindungsgemäß werden die Splenopentin-Derivate derart hergestellt, daß bei Anwendung entsprechender Schutzgruppen sowie unterschiedlicher Acylierungsreagentien eine selektive Acylierung der N-Positionen des Arginins und/oder des Lysins und/oder der O-Position des Tyrosins erreicht und die Bildung von Nebenprodukten vermieden wird.

Verfahren zur Acylierung von Splenopentin sind bisher nicht beschrieben worden. Es hat sich gezeigt, daß die Verwendung üblicher Acylierungsmittel, wie Acetanhydrid und Säurechlorid,in erheblichem Ausmaß zur Bildung unerwünschter Nebenprodukte (Racemisierung am Val und am Tyr, Glutarimid-Bildung) führt; bei der Herstellung von $N^\alpha$-Arg- und $N^\epsilon$-Lys-diacylierten Splenopentinen (DAc-SP5) entstehen O-Acyl-Tyr-Nebenprodukte. Auch die Umsetzung mit Essigsäure-p-nitrophenylester verläuft nicht nebenproduktfrei; zudem bereitet die Entfernung des dabei entstehenden toxischen p-Nitrophenols, speziell aus argininhaltigen Peptiden, erhebliche Schwierigkeiten. Erfindungsgemäß gelingt die selektive Acylierung jedoch bei Verwendung der entsprechenden N-Hydroxynorborn-5-en-2,3-dicarboximidester von Carbonsäuren, so zur Herstellung von Monoacylsplenopentinen (MAc-SP5) bzw. Diacylsplenopentinen (DAc-SP5). Die Herstellung der mono- wie der diacetylierten Acetylverbindungen gelingt vorteilhaft durch Verwendung von N-Acetoxynorborn-5-en-2,3-dicarboximid (Acetyl-ONB) der Formel II

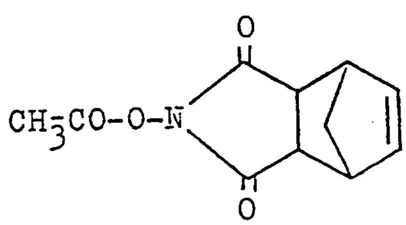

(II)

sowie von 1-Acetoxybenzotriazol (Acetyl-OBt) der Formel III

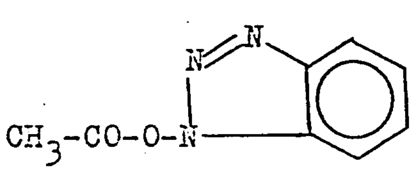

(III).

Überraschenderweise hat es sich gezeigt, daß das überhaupt erstmals als Acetylierungsmittel eingesetzte Acetyl-ONB (II) besondere Vorteile aufweist. Aufgrund seiner hohen Hydrolysebeständigkeit besitzt

es eine sehr gute Lagerstabilität und kann bei Acetylierungen sowohl in organischen als auch in wäßrigen Medien eingesetzt werden, und zwar vor allem wegen seiner hohen Reaktionsfähigkeit mit Aminogruppen auch ohne Basenzusatz und bei extrem kurzen Reaktionszeiten. Das im Reaktionsverlauf entstehende N-Hydroxynorborn-5-en-2,3-dicarboximid läßt sich leicht abtrennen und ist nicht toxisch, so daß seine Anwendung vor allem in der pharmazeutischen Industrie besonders vorteilhaft erscheint.

Mit Hilfe dieser Acylierungsmittel gelingt es, die Mono- und Diacylverbindungen in guten Ausbeuten (90 % d.Th.) unter Vermeidung unerwünschter Nebenreaktionen herzustellen. Bei Durchführung der Acylierung in Gegenwart von tertiären Aminen erhält man die entsprechenden Triacylverbindungen. Die Trennung der erhaltenen acylierten Splenopentine erfolgt besonders vorteilhaft dadurch, daß die chromatographische Trennung an carboxylierten Kationenaustauschern in wäßrigen Elutionssystemen bei einer Ionenstärke zwischen $10^{-2}$ und $10^{-5}$ mol/l durchgeführt wird. Geeignete Trägermaterialien sind Acrylsäure-Divinylbenzol-Copolymerisate. Bei einer für Ionenaustauschchromatographien üblichen hohen Kapazität des Trägers (5 bis 10 mg Peptid/ml Säulenvolumen) erfolgt erfindungsgemäß die Elution der Peptide isokratisch mit wäßrigen Elutionsmitteln, wobei die notwendige Ionenstärke zwischen $10^{-2}$ und $10^{-5}$ mol/l durch Zugabe von Säuren realisiert wird.

$N^{\alpha}$-Arg,$N^{\epsilon}$-Lys,O-Tyr-Triacetylsplenopentin, $N^{\alpha}$-Arg,$N^{\epsilon}$-Lys-Diacetylsplenopentin, $N^{\alpha}$-Arg-Monoacetylsplenopentin, $N^{\epsilon}$-Lys-Monoacetylsplenopentin und die Enantiomeren D-Tyr-$N^{\alpha}$-Arg,$N^{\epsilon}$-Lys-Diacetylsplenopentin und L-Tyr-$N^{\alpha}$-Arg,$N^{\epsilon}$-Lys-Diacetylsplenopentin werden überraschenderweise mit steigendem K'-Wert eluiert und damit entsprechend ihrer Polarität getrennt. Die Wiederfindungsrate für alle getrennten Peptide ist > 95 %.

Besonders gute Trenneffekte werden erreicht, wenn das Elutionsmittel 10 bis 20% Ethanol enthält. Auf Grund der geringen Ionenstärke im Elutionssystem können die Peptide durch Einengen der entsprechenden Fraktionen und anschließende Gefriertrocknung einfach und schonend mit einer durch analytische RP-HPLC bestimmten Reinheit > 98 % isoliert werden. Bei anderen Verfahren notwendige zusätzliche Entsalzungsschritte sind nicht erforderlich. Weitere Vorteile des erfindungsgemäßen Verfahrens sind der Einsatz kostengünstiger Chromatographieträgermaterialien und die hohe Wiederverwendbarkeit des Trägers(> 100 Chromatographiezyklen).

Durch Vermischen der so erhaltenen neuen Verbindungen mit pharmazeutisch akzeptablen Trägerstoffen und/oder physiologisch verträglichen Verdünnungsmitteln gelangt man zu den entsprechenden pharmazeutischen Verabreichungsformen.

Mit der Acylierung von Splenopentin wird das Ziel erreicht, die enzymatische Abbaustabilität zu erhöhen und damit die Bioverfügbarkeit des Wirkstoffes zu verbessern.

Zum Nachweis der immunologischen Wirkungen wurden zunächst folgende Modelle verwendet:

- Die quantitative Zunahme von T4-Antigenen auf der Oberfläche menschlicher T-Lymphocyten bei Patienten mit Systemischem Lupus Erythematodes (SIE) in vitro (Beispiel 8, Tab. 1) und
- die Antikörperbildung gegen Schafserythrocyten (Plaque-bildende Milzzellen) durch AB/Bln.-Mäuse nach Röntgenbestrahlung (Beispiel 8, Tab. 2).

  Die erfindungsgemäß hergestellten acylierten Splenopentine ( Ac-SP5 ) weisen weitere günstige pharmakologische Eigenschaften, insbesondere solche zur Normalisierung des Immunsystems, auf. Daher eignen sie sich günstig zur Anwendung bei folgenden medizinischen Indikationen:

- Behandlung viraler Infektionen (Beispiel 13, Tab. 3.1.-3.3.). Die Ac-SP5 haben sich als geeignete Mittel gegen Influenzaviren sowie als Mittel zur Pankreatitis-Behandlung nach Infektion mit Coxsackieviren erwiesen. Hinsichtlich des Ausmaßes der Stimulierung der antiviralen Immunabwehr ist festgestellt worden, daß bei Gabe von Virusmengen, die zu einer Infektion mittlerer Schwere führen, die Erkrankung vollständig verhindert werden kann und daß die zur Auslösung einer Erkrankung gleichen Schweregrades notwendige Virusdosis unter Behandlung etwa 10fach höher ist als bei Individuen ohne Therapie.
- Behandlung nach Chemotherapie (Beispiel 14, Abb. 4.1.,Tab.4.1.) Die Mittel sind geeignet zur Überwindung immunsuppressiver Zustände nach chemotherapeutischen Maßnahmen, z. B. bei der Krebsbehandlung oder infolge Überdosierung von Cyclosporin.

  Eine Normalisierung der humoralen Immunantwort nach Cyclophosphamid- oder Dexamethason-Behandlung von Mäusen läßt sich durch Gabe von Ac-SP5 erreichen. Die Ac-SP5 haben jedoch keinen Einfluß auf die Ausbildung der normalen Antigen-spezifischen humoralen Immunantwort, so daß nachteilige immunomodulatorische Nebenwirkungen nicht zu erwarten sind (Abb. 4.1.).

  Die Ac-SP5 bewirken eine beschleunigte Rekonstitution des Immunsystems von Patienten mit sekundärer Immundefizienz als Folge immunsuppressiver Therapie (Tab. 4.1.).

- Stimulierung des Wachstums und der Reifung von Knochenmarkzellen (Beispiel 15, Tab. 5.1.-5.2., Abb. 5.1.).

Die Anwendung der erfindungsgemäßen Verbindungen führt in vitro zu einer Erhöhung der Bildungsrate von Stammzellkolonien aus Maus-Knochenmarkzellen, die mit Leukocyten von Normalpersonen oder SIE-Patienten in Agar ko-kultiviert wurden. Die vermehrte Bildung von "Kolonien" als Folge der Gegenwart von Ac-SP5 ist ein Hinweis dafür, daß durch Ac-SP5 direkt die Proliferation unreifer Stammzellen des Knochenmarks angeregt wird und/oder Leukocyten des Menschen unter dem Einfluß von Ac-SP5 vermehrt Kolonie-bildende Faktoren sezernieren (Tab. 5.1.).

Bei subletal bestrahlten Mäusen, denen syngene Knochenmarkzellen transplantiert wurden, ist eine beschleunigte Rekonstitution der humoralen Immunantwort zu beobachten (Abb. 5.1.). Die Wirkstoffe sind bei immunsuppressiver bzw. cytostatischer Therapie, nach Bestrahlung und nach Knochenmarktransplantationen anwendbar. Sie wirken dadurch, daß sie spezifische Bindungsorte (Rezeptoren) auf Knochenmarkzellen des Menschen (insbesondere auf Stammzellen) besetzen, die durch Radioligand-Bindungsstudien nachgewiesen verden können. Derartige Bindungsstellen finden sich auch auf Thymocyten und können durch $^{3}$H-markiertes Splenopentin oder durch $^{125}$J-markiertes DAc-SP5 nachgewiesen werden (Tab. 5.2.).

- Therapie von HIV-Infektionen (Beispiel 16, Tab. 6.1. und Abb. 6.1.).

DAc-SPS-Gabe bewirkt bei HIV-infizierten Patienten eine Anhebung der Gesamtlymphocytenzahl in den Kontrollbereich sowie eine Erhöhung der Absolutzahl von $CD3^{+}$-, $CD4^{+}$- und $DR^{+}$-Zellen sowie eine Verbesserung des $CD4^{+}/CD8^{+}$-Verhältnisses. Nach 16wöchiger Therapie traten keine Nebenwirkungen auf (Tab. 6.1.).

Eine Kombination mit dem in der AIDS-Therapie eingesetzten Cytostaticum und Revertasehemmer Azidothymidin sowie wirksameren Substanzen dieses Typs (Fluothymidin) führt zu einer beschleunigten Normalisierung der durch diese Substanzen bewirkten nachteiligen Veränderungen im Immunsystem (Abb. 6.1.).

- Verhinderung immunsuppressiver Effekte chronischer Intoxikationen (Beispiel 17, Tab. 7).

Die Wirkung der Ac-SP5 besteht darin, daß die durch Alkohol und andere chronisch wirkende Noxen induzierte Suppression der Antigen-spezifischen humoralen Immunreaktion sowie die Verminderung der Zahl bzw. Aktivität phagocytierender Zellen aufgehoben und die partielle Atrophie des Thymus und der Milz verhindert wird.

- Therapie von Autoimmunreaktionen (Beispiel 18, Tab. 8.1.-8.2.).

Die erfindungsgemäßen Substanzen eignen sich zur Therapie verschiedener Krankheiten mit einer Autoimmunkomponente (dargestellt am Beispiel der induzierten Autoimmunreaktion gegen Neuroantigene (Tab. 8.1)), wie der Rheumatoiden Arthritis, von SLE sowie von atopischen Reaktionen. Sie bewirken jedoch keine nachteilige Reduktion des Antigen-spezifischen Antikörpertiters nach erfolgter Immunisierung (Tab. 8.2.).

Die Erfindung wird im folgenden anhand von Ausführungsbeispielen näher erläutert.

## Beispiel 1

Herstellung von Arginyl($N^{\epsilon}$-acetyl-lysyl)glutamylvalyl)tyrpsin ($N^{\epsilon}$-MAc-SP5)

1.1 Acylierung

1,83 g (2 mmol) BOC-Arg-Lys-Glu-Val-Tyr-OH•2HOAc werden in 5 ml Dimethylformamid gelöst und mit 0,487 g (2,2 mmol) Acetyl-ONB umgesetzt. Nach 1 Stunde wird die Reaktionsmischung in 1N HCl eingetragen; das ausgefallene Produkt wird abgesaugt und mit Wasser neutral gewaschen. Nach Umfällen aus Methanol/Essigester werden 1,72 g (Ausbeute 96,3 % d.Th.) BOC-Arg-Lys(Ac)-Glu-Val-Tyr-OH•HOAc erhalten.

1.2 Schutzgruppenabspaltung

1,34 g (1,5 mmol) BOC-Arg-Lys(Ac)-Glu-Val-Tyr-OH•HOAc werden 30 min mit 10 ml Trifluoressigsäure/Dichlorethan (1:1) behandelt und nach Abdestillieren der überschüssigen Trifluoressigsäure aus Methanol mit Ether gefällt.
Ausbeute: 1,34 g (92,8 % d.Th.).
Arg-Lys(Ac)-Glu-Val-Tyr-OH • 2 TFA
Durch Lyophilisieren aus verdünnter Salzsäure wird das entsprechende $N^{\epsilon}$-MAc-SP5•2HCl erhalten.
Molekülmasse 809,76. Summenformel $C_{33}H_{56}N_{9}O_{10}Cl_{2}$.

**Beispiel 2**

Herstellung von Arginyl-($N^{\epsilon}$-Palmitoyl-lysyl)-glutamylvalyltyrosin ($N^{\epsilon}$-Pal-SP5)

2.1 Acylierung

1,83 g (2 mmol) BOC-Arg-Lys-Glu-Val-Tyr-OH•2HOAc werden in 5 ml DMF gelöst und mit 0,919 g (2,2 mmol) Pal-ONB umgesetzt. Die Aufarbeitung und Reinigung erfolgt analog 1.1.
Ausbeute an BOC-Arg-Lys(Pal)-Glu-Val-Tyr-OH•HOAc 2,07 g (94,6 d.Th.).

2.2 Schutzgruppenabspaltung

1,64 g (1,5 mmol) BOC-Arg-Lys(Pal)-Glu-Val-Tyr-OH•HOAc werden 30 min mit 10 ml Trifluoressigsäure/Dichlorethan (1:1) behandelt und nach Abdestillieren der überschüssigen Trifluoressigsäure aus Methanol mit Ether gefällt.
Ausbeute an Arg-Lys(Pal)-Glu-Val-Tyr-OH•2TFA 1,63 g (93,7 % d.Th.).

Durch Lyophilisieren aus verdünnter Salzsäure wird das entsprechende $N^{\epsilon}$-Pal-SP5•2HCl erhalten.
Molekülmasse 1090,2. Summenformel $C_{48}H_{92}N_9O_{14}Cl_2$.

**Beispiel 3**

Herstellung von ($N^{\alpha}$-Acetyl-arginyl)lysylglutamylvalyltyrosin ($N^{\alpha}$-MAc-SP5)

3.1 Acylierung

1,16 g (1 mmol) Arg($NO_2$)-Lys(Z)-Glu(OBzl)Val-Tyr-OBzl•TFA werden in 5 ml Dimethylformamid gelöst und nach Zugabe von 180 µl N-Methylmorpholin (oder eines anderen tertiären Amins) mit 0,265 g (1,2 mmol) N-Acetoxynorborn-5-en-2,3-dicarboximid umgesetzt.

Nach 30 min wird die Reaktionsmischung unter Rühren in 1N HCl eingetragen; der Niederschlag wird abgesaugt und mit Wasser gewaschen. Das Produkt wird in etwa 10 ml Dimethylformamid gelöst und mit 5 %iger $NaHCO_3$-Lösung gefällt, filtriert und mit Wasser gewaschen. Nach Umfällung aus Methanol/Ether werden 1,05 g (Ausbeute 96,2 % d.Th.) geschütztes $N^{\alpha}$-Acetylsplenopentin erhalten.

3.2 Schutzgruppenabspaltung

Das geschützte Acetylpeptid wird in 90 %iger Essigsäure suspendiert und in Gegenwart von Palladiumschwarz hydriert.
Ausbeute an $N^{\alpha}$-Acetyl-SP5•2HOAc 760,8 g (95,6 % d.Th.).
Molekülmasse 856,94. Summenformel $C_{37}H_{62}N_9O_{14}$.

**Beispiel 4**

Herstellung von ($N^{\alpha}$-Acetyl-arginyl)($N^{\epsilon}$-acetyl-lysyl)glutamylvalyltyrosin (DAc-SP5)

4.1 Synthese von DAc-SP5 durch Umsetzung von SP5 mit N-Acetoxynorborn-5-en-2,3-carboximid (II)

43,7 g (50 mmol) Arg-Lys-Glu-Val-Tyr-OH•3HOAc (SP5) werden in 150 ml Wasser gelöst und mit einer Lösung von 33,2 g (150 mmol) Acetyl-ONB in 100 ml Dimethylformamid versetzt. Man gibt dann so lange Dimethylformamid zu, bis eine homogene Lösung entsteht. Nach einstündigem Rühren bei Raumtemperatur (DC-Kontrolle) wird das Lösungsmittel im Vakuum entfernt und der verbleibende Rückstand mit Ether oder Essigester verrieben. Der Feststoff wird abgesaugt, mit Ether gewaschen und im Vakuum über KOH getrocknet.
Ausbeute an DAc-SP5•HOAc: 38,54 g (92 % d.Th.).

Durch Lyophilisieren aus verdünnter Salzsäure wird das entsprechende DAc-SP5•HCl•$2H_2O$ erhalten.

| Molekülmasse 850,39. Summenformel $C_{35}H_{60}N_9O_{13}Cl$. | | | |
|---|---|---|---|
| Analyse: | C (%) | H (%) | N (%) |
| ber.: | 49,43 | 7,11 | 14,83 |
| gef.: | 48,62 | 7,26 | 14,00. |

4.2 Synthese von DAc-SP5 durch Umsetzung von SP5 mit 1-Acetoxybenzotriazol (Acetyl-OBt) (III)

Die Umsetzung von SP5 mit Acetyl-OBt und die Aufarbeitung erfolgen analog 4.1.
Ausbaute: 90 % d.Th.

**Beispiel 5**

Herstellung von ($N^{\alpha}$-Palmitoyl-arginyl)lysylglutamylvalyltyrosin ($N^{\alpha}$-Pal-SP5)

5.1 Acylierung

1,16 g (1 mmol) H-Arg($NO_2$)-Lys(Z)-Glu(OBzl)-Val-Tyr-OBzl•TFA werden in 5 ml Dimethylformamid gelöst und nach Zugabe von 120 µl N-Methylmorpholin (oder eines anderen tertiären Amins) mit 0,626 g (1,2 mmol) Palmitinsäure-(N-oxy-norborn-5-en-2,3-dicarboximid)-ester umgesetzt.

Nach Aufarbeitung analog 3.1 und Umfällung aus Methanol/Ether werden 1,21 g (Ausbeute: 94,3 % d.Th.) geschütztes $N^{\alpha}$-Palmitoylsplenopentin erhalten.

5.2 Schutzgruppenabspaltung

Das geschützte Palmitoylpeptid wird in 90 %iger Essigsäure suspendiert und in Gegenwart von Palladiumschwarz hydriert.
Ausbeute an $N^{\alpha}$-Pal-SP5•2HOAc: 920 mg (92,7 % d.Th.).
Molekülmasse 1035,29. Summenformel $C_{51}H_{88}O_{13}N_9$.

**Beispiel 6**

Herstellung von N-Acetoxynorborn-5-en-2,3-dicarboximid (Acetyl-ONB)

17,92 g (0,1 mol) N-Hydroxynorborn-5-en-2,3-dicarboximid werden in 100 ml Chloroform weitgehend gelöst und bei 0 °C unter Rühren mit 21 ml (0,15 mol) Triethylamin und danach mit 19 ml (0,2 mol) Acetanhydrid versetzt. Nach zweistündigem Rühren bei Raumtemperatur wird das Lösungsmittel weitgehend entfernt, mit Wasser versetzt und der Niederschlag abfiltriert.

Nach Umkristallisieren aus Isopropanol/Wasser werden 19,33 g (Ausbeute 87,4 % d.Th.) Acetyl-ONB erhalten.
F. 112,5 bis 113,5 °C.

**Beispiel 7**

Herstellung von 1-Acetoxybenzotriazol (Acetyl-OBt)

13,51 g (0,1 mol) 1-Hydroxybenzotriazol werden in 100 ml Chloroform gelöst. Nach Zugabe von 16,8 ml (0,12 mol) Triethylamin wird auf 0 °C abgekühlt, unter Rühren mit 19 ml (0,2 mol) Acetanhydrid versetzt und 2 Stunden bei Raumtemperatur nachgerührt.Der nach Entfernen des Lösungsmittels verbleibende Feststoff wird aus Essigester oder Benzol umkristallisiert.
Ausbeute 14,88 g (84 % d.Th.).
F. 96 bis 98 °C.

**Beispiel 8**

Immunbiologische Wirkung von $N^{\epsilon}$-MAc-SP5

$N^{\epsilon}$-MAc-SP5 (Sequenz 32 bis 36 des Milzhormons Splenin) hat die gleichen bzw. ähnliche immunbiologische Wirkungen wie das Gesamtmolekül Splenin. So induziert $N^{\epsilon}$-MAc-SP5 die quantitative Zunahme von T4-Antigenen auf der Oberfläche von T-Lymphocyten, die auf der Oberfläche funktionstüchtiger T-Helferlymphocyten nachweisbar sind. Funktionstüchtige T-Helferlymphocyten stimulieren die Antikörperbildung. Zum Nachweis einer Erhöhung der Antikörper-Bildung durch $N^{\epsilon}$-MAc-SP5 wurde ein relevantes tierexperimentelles Modell gewählt: AB/Bln.-Mäuse wurden mit Röntgenstrahlen subletal bestrahlt und danach mit bzw. ohne $N^{\epsilon}$-MAc-SP5 therapiert. Gleichzeitig erfolgte die Immunisierung der Tiere mit Schafserythrocyten. Da subletal bestrahlte Tiere die Fähigkeit verlieren, quantitativ ausreichend Antikörper zu produzieren, ist eine vermehrte Bildung von Antikörpern gegen Schafserythrocyten bei den mit $N^{\epsilon}$-MAc-SP5 behandelten Tieren ein Maß für die Regeneration des Immunsystems durch $N^{\epsilon}$-MAc-SP5. Mit $N^{\epsilon}$-MAc-SP5 behandelte Tiere konnten bereits nach 34 Tagen Antikörper in gleicher Menge wie unbestrahlte Kontrolltiere bilden. Im Vergleich dazu waren nicht mit $N^{\epsilon}$-MAc-SP5 behandelte Tiere dazu erst 48 Tage nach der einmaligen Röntgenbestrahlung befähigt.

Tests

8.1 Zunahme von T4-Antigenen auf der Oberfläche menschlicher Lymphocyten nach Kontakt mit $N^{\epsilon}$-MAc-SP5

Funktionstüchtige (reife) T-Helferlymphocyten besitzen auf ihrer Oberfläche T4-Antigene, T-Suppressorlymphocyten T8-Antigene. Bei Patienten mit Systemischem Lupus Erythematodes (SLE) wurde mittels monoklonaler Antikörper ein verminderter Gehalt von T4-Antigenen auf der Lymphocytenoberfläche bestimmt.

Als Folge therapeutischer Maßnahmen normalisieren sich die Werte. Wurden vor Therapie die Lymphocyten der Patienten mit $N^{\epsilon}$-MAc-SP5 in Kontakt gebracht, war nach 2stündiger Inkubation eine individuell unterschiedliche, jedoch permanent erhöhte Anzahl von T4-Antigenen nachweisbar, wie aus Tabelle 1 hervorgeht.

## Tabelle 1

Quantitative Zunahme von T4-Antigenen auf der Oberfläche menschlicher T-Lymphocyten nach Kontakt mit $N^{\epsilon}$-MAc-SP5. Bestimmung des T4-Antigens mittels monoklonaler Antikörper (VI$^{T4}$-CD4 cluster und VI$^{TB}$-CDB cluster). T-Lymphocyten von Patienten mit Systemischem Lupus Erythematodes (SLE).

| Patient Nr. | | T-Zellen-Oberflächenantigene (%) T4 | TB |
|---|---|---|---|
| 1 | Kontrolle (K) (ohne $N^{\epsilon}$-MAc-SP5) | 1 | 10 |
| | + $N^{\epsilon}$-MAc-SP5 | 54 | 10 |
| 2 | K | 48 | 40 |
| | + $N^{\epsilon}$-MAc-SP5 | 60 | 45 |
| 3 | K | 44 | 50 |
| | + $N^{\epsilon}$-MAc-SP5 | 66 | 48 |
| 4 | K | 42 | 12 |
| | + $N^{\epsilon}$-MAc-SP5 | 65 | 15 |
| 5 | K | 45 | 40 |
| | + $N^{\epsilon}$-MAc-SP5 | 67 | 42 |

Die TB-Antigene (Oberflächenantigene auf T-Suppressorlymphocyten bzw. cytotoxischen T-Lymphocyten) veränderten sich dagegen quantitativ nicht. T4-Antigene nehmen zu, wenn sich T4-Zellen vermehren bzw., wenn die Antigene vermehrt auf ihrer Oberfläche exprimiert werden. Die Untersuchungen ergaben, daß die Lymphocyten der SLE-Patienten nach Kontakt mit $N^{\epsilon}$-MAc-SP5 keine erhöhten $^{3}$H-Thymidineinbauraten aufwiesen (910 ± 180 Impulse je Minute ohne $N^{\epsilon}$-MAc-SP-5-Kontakt/820 ± 210 Impulse je Minute nach Kontakt mit $N^{\epsilon}$-MAc-SP5; n = 5, keine signifikante Abweichung im t-Test). Es ist daher anzunehmen, daß die Zunahme der T4-Antigene nicht durch eine Vermehrung von T4-Zellen zustandekommt, sondern Folge einer strukturellen Veränderung der T-Zelloberfläche, möglicherweise identisch mit einem "Reifeprozeß" der Zelle, ist.

Reife, funktionstüchtige T-Helferlymphocyten stimulieren die Antikörperbildung durch B-Lymphocyten. Daher wurde untersucht, ob sich nach systemischer $N^{\epsilon}$-MAc-SP5-Applikation die Antikörperbildung gegen definierte Antigene (Schafserythrocyten) erhöht.

8.2 Antikörperbildung nach Immunosuppression und systemischer $N^{\epsilon}$-MAc-SP5-Medikation

Zum Nachweis der Beeinflussung der Antikörperbildung wurde ein relevantes tierexeperimentelles Modell verwendet: Vier Monate alte AB/Bln.-Mäuse (Akademie der Wissenschaften der DDR, Berlin-Buch) wurden systemisch (subletal) mit Röntgenstrahlen behandelt (600 c Gy) und anschließend mit bzw. ohne $N^{\epsilon}$-MAc-SP5 therapiert (Tiergruppen Nr. 1, 2 und 3 der Tabelle 2). Eine weitere Tiergruppe wurde weder röntgenbestrahlt noch mit $N^{\epsilon}$-MAc-SP5 behandelt (Gruppe Nr. 4, Tabelle 2). Die Tiergruppen Nr. 2 und 3, wurden umtägig mit entweder 10 µg oder 50 µg $\epsilon$-MAc-SP5 behandelt (intraperitoneale Applikation). In unterschiedlichen Zeitabständen nach Bestrahlung (14. Tag, 20. Tag usw., Tabelle 2) wurde mittels des

Jerne-Plaque-Testes (modifiziert nach Eckert, R., Pfüller, U., Kindt, A., Reichelt, E., und Franz, H.: Histaminrezeptor-tragende Lymphocyten. III. Suppression von Immunreaktionen nach Abtöten der Histaminrezeptor-tragenden Lymphozyten durch ein Konjugat aus Histamin und der A-Kette des Mistellektins I. Biomed. Biochem. Acta 44 (1985) 1239-1246) das Ausmaß der Antikörperbildung der Tiere gegen Schafserythrocyten bestimmt. Dazu wurden die Tiere 5 Tage vor der Untersuchung mit Schafserythrocyten immunisiert (intraperitoneale Applikation von je 5 x $10^8$ Zellen pro Tier) und an den in Tabelle 2 angegebenen Untersuchungstagen hinsichtlich ihrer Fähigkeit, Antikörper gegen Schafserythrocyten zu bilden, untersucht (Methodische Details siehe Diezel, W., et al., Biomed. Biochim. Acta 45 (1986) 1349 bis 1352).

Tabelle 2:

Antikörperbildung gegen Schaferythrocyten (Plaque-bildende Milzzellen) durch AB/Bln.-Mäuse nach einmaliger Röntgenbestrahlung der Tiere mit 600 cGy und nachfolgender Behandlung mit $N^{\epsilon}$-MAc-SP5. Umtägige Peptidapplikation von 10 µg und 50 µg pro Tier. Jeder Wert (Mittelwert $\pm$ Streuung) repräsentiert den Mittelwert von mindestens 5 Tieren.

| Nr. | Behandlung | Anzahl Plaque-bildender Milzzellen pro Milz | | | | | |
|---|---|---|---|---|---|---|---|
| | | Tag 14 | Tag 20 | Tag 27 | Tag 34 | Tag 41 | Tag 48 |
| 1. | Röntgenbestrahlung | $1600 \pm 600$ | $1500 \pm 500$ | $4000 \pm 1000$ | $12000 \pm 2500$ | $28000 \pm 7800$ | $40000 \pm 6600$ |
| 2. | Röntgenbestrahl.[1)] + MAc-SP 5 (10 µg) | $3200 \pm 1500$ | $4400 \pm 1000$ | $17000 \pm 2000$ | $41000 \pm 9500$ | $44000 \pm 8500$ | $44500 \pm 4000$ |
| 3. | Röntgenbestrahlung + MAc-SP 5 (50 µg) | $3000 \pm 400$ | $5200 \pm 900$ | $15000 \pm 3000$ | $39000 \pm 7000$ | n.b. [2)] | n.b. |
| 4. | ohne Röntgenbestr. ohne MAc-SP 5 | $44100 \pm 6600$ | $42500 \pm 4000$ | $44000 \pm 5500$ | $44000 \pm 8000$ | $43000 \pm 9300$ | $43500 \pm 9000$ |

1) U-Test (Mann und Whitney). Vergleich zwischen Nr.1/2 und 1/3: Tag 14: p 0,05; Tag 20: p 0,001; Tag 34: p 0,001; Vergleich Nr. 1/2: Tag 41: p 0,01; Tag 48: kein signifikanter Unterschied. Vergleich 2/4 und 3/4: Tag 34: kein signifikanter Unterschied.

2) n.b. = nicht bestimmt.

Es ist bekannt, daß subletal bestrahlte Tiere die Fähigkeit verlieren, ausreichend Antikörper zu bilden. Eine normale Bildung tritt erst nach einer gewissen Latenzperiode ein, in welcher sich die antikörperbildenden Zellen der Tiere regenieren. In den Experimenten konnten die Tiere frühestens 48 Tage nach der einmaligen Röntgenbestrahlung Antikörper in gleicher Menge wie unbestrahlte Kontrolltiere bilden (kein signifikanter Unterschied zwischen Nr. 1 und 4 am Tag 48, Tabelle 2). Im Vergleich dazu waren mit $N^{\epsilon}$-MAc-SP5 behandelte Tiere schon 34 Tage nach der Röntgenbestrahlung zu einer normalen Antikörperproduktion

befähigt (kein signifikanter Unterschied zwischen den Werten Nr. 2 und 4 bzw. 3 und 4 am Tag 34, Tabelle 2).

Die vorgestellten Ergebnisse berechtigen zu der Annahme, daß Immunabwehrschwächen durch $N^{\epsilon}$-MAc-SP5 günstig beeinflußt werden können. Ein derartiger Zustand ist angeboren bzw. erworben. Eine erworbene Immunabwehrschwäche kann als Folge notwendiger therapeutischer Maßnahmen (Zytostatikatherapie und Bestrahlungstherapie bei Krebserkrankungen) auftreten bzw. ist obligat bei AIDS im Spätstadium der Krankheit. Entsprechend der vorgestellten Ergebnisse sollte durch $N^{\epsilon}$-MAc-SP5 eine derartige beeinträchtigte Funktion des Immunsystems zumindest partiell behoben werden können.

## Beispiel 9

Trennung von Mono-, Di- und Triacetylsplenopentinen (Mono-, Di- und Triacetyl-SP5 ; $N^{\alpha}$-Acetyl-arginylsplenopentin (Monoacetyl($N^{\alpha}$-Arg)-SP5); $N^{\alpha}$-Arg,$N^{\epsilon}$-lys-Diacetylsplenopentin (Diacetyl($N^{\alpha}$-Arg,$N^{\epsilon}$-Lys)-SP5); $N^{\alpha}$-Arg,$N^{\epsilon}$-lys, O-Tyr-Triacetylsplenopentin (Triacetyl($N^{\alpha}$ -Arg,$N^{\epsilon}$ -Lys,O-Tyr)-SP5)).

Auf eine Säule von 150 mm Länge und 9 mm Durchmesser (V = 9,54 ml), die als Trägermaterial einen carboxylierten Kationenaustauscher einer Korngröße von 50 bis 90 µm enthält, werden 50 mg eines äquimolaren Gemisches von Mono-, Di- und Triacetyl-SP5 aufgegeben.

Die Elution der Substanzen erfolgt bei einer Fließgeschwindigkeit von 5 ml/h mit 11,5 % Ethanol von pH 2,45 (Essigsäure). Die Substanzen werden in der Reihenfolge Mono-, Di- und Triacetyl-SP5 mit R > 1 und einer Wiederfindungsrate von > 95 % je Substanz getrennt.

## Beispiel 10

Trennung von $N^{\alpha}$ -Acetyl-arginylsplenopentin (Monoacetyl($N^{\alpha}$ -Arg)-SP5) und $N^{\epsilon}$-Acetyl-lysylsplenopentin (Monoacetyl($N^{\epsilon}$-Lys)-SP5).

Auf eine Säule von 200 mm Länge und 9 mm Durchmesser (V = 12,7 ml), die als Trägermaterial einen carboxylierten Kationenaustauscher einer Korngröße von 40 bis 50 µm enthält, wird ein äquimolares Gemisch von Monoacetyl($N^{\alpha}$-Arg)-SP5 und Monoacetyl($N^{\epsilon}$-Lys)-SP5 aufgegeben. Die Elution erfolgt mit einem wäßrigen Laufmittel von pH 2,35 (HCl) bei einer Fließgeschwindigkeit von 10 ml/h. Die Substanzen werden entsprechend ihrer Polarität (1. Monoacetyl($N^{\alpha}$-Arg)-SP5; 2. Monoacetyl($N^{\epsilon}$-Lys)-SP5 mit einer Wiederfindungsrate von 97 % eluiert.

Die Auflösung beträgt 1,5.

## Beispiel 11

Trennung von $N^{\alpha}$-Arg,$N^{\omega}$-Arg,$N^{\epsilon}$-lys-Triacetylsplenopentin (Triacetyl($N^{\alpha}$-Arg,$N^{\omega}$-Arg,$N^{\epsilon}$-Lys)-SP5) und $N^{\alpha}$-Arg,$N^{\epsilon}$-lys-Diacetylsplenoplentin (Diacetyl($N^{\alpha}$-Arg,$N^{\epsilon}$-Lys)-SP5).

Auf eine Säule von 200 mm Länge und 5 mm Durchmesser, die als Träger einen carboxylierten Kationenaustauscher einer Korngröße von 50 bis 60 µm enthält, wird ein Gemisch von je 5 mg beider Peptide aufgegeben. Die Elution erfolgt bei einer Fließgeschwindigkeit von 3 ml/h mit 11,5 % Ethanol, von pH 2,57 (Essigsäure). Es wird eine Auflösung von 0,8 bei einer Wiederfindungsrate von 96 % pro Substanz erreicht.

## Beispiel 12

Trennung eines Syntheserohproduktes acylierter Splenopentine

Enthaltene Substanzen:

1. Monoacetyl($N^{\alpha}$-Arg)-SP5
2. Diacetyl($N^{\alpha}$-Arg, $N^{\epsilon}$-Lys)-SP5
(Hauptprodukt, Anteil 75% nach HPLC)
3. Triacetyl($N^{\alpha}$Arg, $N^{\omega}$-Arg, $N^{\epsilon}$-Lys)-SP5
4. Triacetyl($N^{\alpha}$-Arg, $N^{\epsilon}$-Lys-O-Tyr)-SP5
5. Diacetyl($N^{\alpha}$-Arg, $N^{\epsilon}$-Lys, D-Tyr)-SP5.

Auf eine Säule von 60 cm Länge und 10 cm Durchmesser, die 4,5 l eines carboxylierten Kationenaustauschers einer Korngröße von 50 bis 90 µm enthält, werden 35 g eines Splenopentin-Syntheserohproduktes in 150 ml Elutionslösung aufgetragen. Die Trennung des Substanzgemisches erfolgt durch isokratische Elution mit 10 % Ethanol von pH 2,5 (Essigsäure) bei einer Fließgeschwindigkeit von 80 ml/min. Das

fraktionierte Eluat wird bei 276 mm vermessen. Anschließend vereinigt man die Peakvolumina, engt bis zur Trockne ein und lyophilisiert unter Zusatz äquimolarer Mengen Salzsäure.
Gesamtwiederfindungsrate: 95,6 %;
Hauptprodukt: 19 g (54,3 %);
HPLC Hauptprodukt: 98,5 % Reinheit.

**Beispiel 13:** Behandlung viraler Infektionen

Tab. 3.1

| Wirkung der DAc-SP5 Behandlung auf die Sterberate von Balb/c-Mäusen nach Infektion (intranasal) mit dem Influenzavirus A/PR/8/34 (n = 10-12) | | | | | |
|---|---|---|---|---|---|
| Infektionsdosis ($EID_{50}$) | $10^1$ | $10^2$ | $10^3$ | $10^4$ | $10^5$ |
| Sterberate nach 6 Tagen in % | | | | | |
| Kontrolle | 50 | 66,6 | 100 | 100 | 100 |
| DAc-SP5-Therapie während der Infektion$^+$ | 33,3 | 33,3 | 66,6 | 100 | 100 |
| DAc-SP5 vor der Infektion$^{++}$ | n.g. | n.g. | n.g. | 100 | 100 |

$^+$ 0,5 mg DAc-SP5, s.c. (pro kg), tägliche Gabe über 5 Tage, Beginn der Behandlung am Tage der Infektion.
$^{++}$ 0,5 mg DAc-SP5 pro kg, s.c., 3 x pro Woche während der 2 Wochen Vor Infektionsbeginn.
n.g. = nicht getestet

Tab. 3.2

| Wirkung der DAc-SP5 Behandlung auf die Entwicklung einer histologisch gesicherten Pankreatitis nach Infektion mit dem Coxsackievirus B4 (Balb/c-Mäuse) (n = 10-25) | | | | |
|---|---|---|---|---|
| Tiere mit Pankreatitis (in %, n = 10-15) | Infektionsdosis ($TCID_{50}$) | | | |
| | $10^3$ | $10^4$ | $10^5$ | $10^6$ |
| Kontrolle | 60 | 60 | 100 | 100 |
| DAc-SP5-Therapie während der Infektion$^+$ | 0 | 0 | 60 | 100 |
| DAc-SP5-Therapie vor der Infektion$^{++}$ | n.g. | 80 | 100 | 100 |

$^+$ 0,5 mg DAc-SP5, s.c. (pro kg), tägliche Gabe über 5 Tage, Beginn der Behandlung am Tage der Infektion.
$^{++}$0,5 mg DAc-SP5 pro kg, s.c., 3 x pro Woche während der 2 Wochen vor Infektionsbeginn.
n.g. = nicht getestet

Tab. 3.3

| Wirkung der DAc-SP5 Behandlung auf die Entwicklung histopathologischer Veränderungen der Lunge bei der Maus (Balb/c) und beim syrischen Hamster (n = 9-10) | | | |
|---|---|---|---|
| | Anzahl der Tiere mit histopathologischem Lungenbefund (%) | | |
| | Kontrolle | DAc-SP5-Therapie$^{+}$ während der Infektion | DAc-SP5-Therapie$^{++}$ vor der Infektion |
| Hamster ($TCID_{50}$ = 2 x $10^{3,5}$) | 100 | 10 | 100 |
| Maus (TCID = $10^{3,5}$) | 100 | 0 | 100 |

$^{+}$ 0,5 mg DAc-SP5, s.c. (pro kg), tägliche Gabe über 5 Tage, Beginn der Behandlung am Tage der Infektion.
$^{++}$0,5 mg DAc-SP5 pro kg, s.c., 3 x pro Woche während der 2 Wochen vor Infektionsbeginn.
n.g. = nicht getestet

**Beispiel 14:** Behandlung nach Chemotherapie

Der Einfluß von DAc-SP5 und immunsuppressiv wirkenden Verbindungen auf die Antigen-spezifische IgM-Antwort bei Mäusen wurde untersucht. Die erhaltenen Ergebnisse sind in den Fig.1 und 2 dargestellt.

Figur 1:

Unabhängig von der Applikationsart hat die Gabe der therapeutischen Dosis von DAc-SP5 (3x im Abstand von 24 Stunden, 0,5 mg/kg) keinen siknifikanten Einfluß auf die Stärke und Kinetik der IgM-Antwort von Mäusen gegen Schafserythrocyten.
Tiere: männliche NMRI-Mäuse (Koloniezucht) im Alter von 2-2,5 Monaten.
Methodik des Jerne-Plaquetests entsprechend R. Eckert und G. Pasternak, Acta biol. med. germ. 31: 127-137 (1973), n = 16 (NaCl-Kontrolle), n = 8 (DAc-SP5-Versuche).

Figur 2:

DAc-SP5 rekonstituiert bereits durch wenige aufeinanderfolgende Applikationen die chemisch induzierts Suppression der humoralen Immunantwort.
Die nach einmaliger Gabe von Cyclophosphamid (7,2 mg/Tier, i. p.) ausgelöste nahezu vollständige Suppression der Antigen-spezifischen IgM-Antwort wird durch Behandlung mit DAc-SP5 (0,5 mg/kg, s. c.) an den 4 nachfolgenden Tagen teilweise rekonstituiert.
Die Reduktion der Antigen-spezifischen IgM-Antwort durch Gabe von Dexamethason ( a) = 0,2 mg/Tier, i. p.; b) = 0,08 mg/Tier, i. p.) wird durch DAc-SP5 (0,5 mg/kg, s. c.) Gabe an den zwei nachfolgenden Tagen vollständig rekonstituiert.
* = p 0,05 (Vergleich mit dem entsprechenden Wert ohne DAc-SP5 Behandlung)
n = 6-8 Versuchstiere wie bei Fig. 1.

Tabelle 4.1. Cytofluorimetrischer Nachweis (quantitative Bestimmung) von Monocyten bzw. Lymphocytensubpopulationen mittels monoklonaler Antikörper bei einer Patientin mit sekundärer Immunodefizienz vor und unter Diacetyl-Splenopentin-Medikation und gleichbleibender sonstiger Therapie

| Parameter | Normalwerte | vor Behandlung | Therapie mit DAc-SP5 (Wochen) | | |
|---|---|---|---|---|---|
| | | | 2 | 3 | 4 |
| Lymphocyten (Gpt/l) | 1,5 - 3,5 | 0,28 | 0,84 | 0,74 | 0,83 |
| CD 3 (Gpt/l) | 1,0 - 2,0 | 0,04 | 0,39 | 0,55 | 0,67 |
| CD 4 (Gpt/l) | 0,6 - 1,3 | 0,03 | 0,29 | 0,34 | 0,38 |
| CD 4/ CD 8 | 1,5 - 3,0 | 0,58 | 2,3 | 2,2 | 2,4 |
| $DR^+$-Monocyten (Gpt/l) | 0 -0,6 | 0,02 | 0,08 | 0,26 | 0,60 |
| $DR^+$-Monocyten (%) | 65 - 90 | 16 | 22 | 62 | 72 |

**Beispiel 15:** Stimulierung des Wachstums und der Reifung von Knochenmarkstammzellen

Tabelle 5.1.:

Anzahl der Zell-"Kolonien" nach Kultivierung von Knochenmarkstammzellen von C57 Bl-Mäusen und Leukocyten von Kontrollpersonen bzw. von Kranken mit Systemischem Lupus Erythematodes in Gegenwart unterschiedlicher DAc-SP5 Konzentrationen (Koloniebildungs-Test). Alle Zahlenwerte repräsentieren die prozentualen Mittelwerte von 4 Parallelkulturen, bezogen auf die "Kolonien" ohne Zusatz von DAc-SP5 (Methodik: Schunck, H., et al., Biomed. Biochim. Acta 46 (1987) 581).

| Gruppe | Exp. | Zell-"Kolonien" pro $10^5$ Knochenmarkzellen ohne und nach Kultivierung der Zellen mit DAc-SP5. Prozentuale Angabe, bezogen auf die "Kolonien" ohne Kultivierung mit DAc-SP5 (= 100 %). DAc-SP5-Konzentration pro 1 ml halbfesten Agar. | | | |
|---|---|---|---|---|---|
| | | 1 µg | 2 µg | 10 µg | 20 µg |
| Kontroll- personen | A | 99 | 98 | 114 | 125 |
| | B | 135 | 136 | 147 | 187 |
| Patienten (Systemischer Lupus Erythematodes) | A | 180 | 316 | 270 | 255 |
| | B | 284 | 873 | 642 | 438 |

Tabelle 5.2.

| Nachweis einer durch den unmarkierten Liganden verdrängbaren Bindungsstelle für $^3$H-Splenopentin und $^{125}$I-Diacetyl-Splenopentin durch Radioligand-Bindungsstudien[1)] und Affinitätsberechnung durch computergestützte Affinitätsspektren-Analyse[2)] | | |
|---|---|---|
| Zellart/Spezies | Radioligand-Dissoziationskonstante ($K_D$, M)[3)] | |
| | $^3$H-SP5 (n = 2-5) | $^{125}$I-DAc-SP5 (n = 2-4) |
| Knochenmark/Ratte | $8.7 \pm 2.2 \times 10^{-8}$ | $8.5 \pm 0.5 \times 10^{-9}$ |
| Knochenmark/Mensch | n.g. | $1.2 \pm 0.3 \times 10^{-8}$ |
| Stammzellinie K 564 /Mensch | $12 \times 10^{-8}$ | n.g. |
| Thymuszellen/Ratte | $7.6 \pm 3.5 \times 10^{-8}$ | n.g. |
| Milzzellen/Ratte | keine verdrängbare Bindung | n.g. |
| Peritonealmastzellen Ratte[4)] | $4.3 \pm 1.3 \times 10^{-8}$ | n.g. |

1) Methodik entsprechend H. Repke und C. Liebmann, Membranrezeptoren und ihre Effektorsysteme, Akademie-Verlag, Berlin und VCH Weinheim, (1987), pp. 55 - 101

[2)] Methodik entsprechend H. Repke, Biochim. Biophys. Acta 929 (1987) 47 - 61
[3)] Berechnet aus Werten unterhalb des Sättigungsbereiches
[4)] Methodik der Zellpräparation: H. Repke et al., Agents and Actions 20 (1987) 216 - 218
n.g. = nicht getestet

Die Antikörperbildung gegen Schafserythrocyten (Plaque-bildende Milzzellen) wurde an AB/Bln.-Mäusen nach einmaliger Röngtenbestrahlung der Tiere mit 800 cGy, nachfolgender Transplantation syngener Knochenmarkzellen und anschließender Medikation mit DAc-SP5 untersucht. Die Ergebnisse sind in Fig. 3 dargestellt. Die Medikation erfolgte mit 10 µg DAc-SP5 3 mal wöchentlich (•). Die Ergebnisse ohne DAc-SP5-Behandlung sind mit dem Symbol 0 bezeichnet. Jeder Wert (Mittelwert ± Streuung) repräsentiert den Mittelwert von mindestens 5 Tieren.
Schraffierte Fläche: Plaque-bildende Milzzellen der Tiere ohne Röntgenbestrahlung und ohne DAc-SP-5-Behandlung. Minimal- und Maximalwerte innerhalb der Versuchszeit von 49 Tagen. Immunisierung der Tiere mit Schafserythrocyten 5 Tage vor der Bestimmung der IgM der Plaque-bildenden Milzzellen.

**Beispiel 16:** Therapie von HIV-Infektionen

Die Wirkung von Diacetyl-Splenopentin auf die Beeinflussung der humoralen Immunreaktion durch den HIV-Revertasehemmer 3'-Fluorthymidin-5'-triphosphat (FdTTP) (E.Matthes et al., Biochem. Biophys. Res. Com. 148,1(1987) S. 78-85) wurde untersucht. Die Ergebnisse sind in den Fig. 4 und 5 zusammengefaßt.

Fig. 4

2,5 Monate alten männlichen Balb/c-Mäusen wurde über das Trinkwasser eine Dosis von 1 mg FdTTP täglich über 4 Tage verabreicht. 24 Stunden nach Absetzen der Substanz erfolgte die dreimalige Gabe von DAc-SP5 (0,5 mg/kg, s.c.) im Abstand von 12 Stunden. 12 Stunden nach der letzten DAc-SP5-Behandlung erfolgte die Immunisierung mit Schafserythrocyten (i.p.). Nach weiteren 24 Stunden wurde erneut FdTTP (s.o.) bis zum Töten der Tiere verabreicht. (Plaquetest: 4. Tag nach der Immunisierung).
Gruppe 1: NaCl-Kontrolle (n = 6)
Gruppe 2: FdTTP (n = 6)
Gruppe 3: FdTTP + DAc-SP5 (n = 6).

Fig. 5

Tiere, Methodik und Substanzen wie bei Fig. 4.
Dauer der FdTTP-Behandlung: 11 Tage 24 Stunden nach Absetzen von FdTTP erfolgte die Gabe von DAc-SP5, die im Abstand von 24 Stunden über 5 Tage fortgesetzt wurde.
Gruppe 1: DAc-SP5 Kontrolle (n = 6)
Gruppe 2: FdTTP (n = 6)
Gruppe 3: FdTTP + DAc-SP5 (n = 6)
Gruppe 4: NaCl-Kontrolle (n = 4)
Der Box-und-Whisker-Plot stellt die Daten einer Stichprobe durch 4 Bereiche gleicher Häufigkeit dar. Die Box enthält die mittleren 50% der Stichprobe, der Querstrich ist der Median (bei normalverteilten Daten in der Mitte der Box). Die Striche ober- und unterhalb der Box überstreichen den Rest der Stichprobe.
x = $p < 0,05$ bzw. $p < 0,01$.

Tabelle 6.1.a Cytofluorometrischer Nachweis (quantitative Bestimmung) von Monocyten bzw. Lymphocytensubpopulationen mittels monoklonaler Antikörper bei HIV-infizierten Patienten vor und unter Therapie mit Diacetyl-Splenopentin

| Patienten (Krankheitsstadien) | Parameter | Normalwerte | Vor Behandlung | Therapie mit Splenopentin (Wochen) 4 | 8 | 12 | 16 | 24 [2] |
|---|---|---|---|---|---|---|---|---|
| Nr. 1 (G.Th.) | Lymphocyten (Gpt/l) | 1,5 - 3,5 | 1,44 | 2,25x | 2,22x | 1,88x | 2,02x | 1,53x |
| CDC: IIIb | CD 3 (Gpt/l) | 1,0 - 2,0 | 0,04 | 0,57 | 0,55 | 0,75 | 1,54x | 0,80 |
| | CD 4 (Gpt/l) | 0,6 - 1,3 | 0,10 | 0,17 | 0,17 | 0,28 | 1,01x | 0,29 |
| | CD 4 / CD 8 | 1,5 - 3,0 | 0,21 | 0,32 | 0,26 | 0,58 | 1,85x | 1,50x |
| | $DR^+$-Monocyten (Gpt/l) | 0 - 0,6 | 0,10 | n.b.[3] | 0,28 | n.b. | 0,26 | 0,37 |
| | $DR^+$-Monocyten (%) | 65 - 90 | 29 | n.b. | 65x | n.b. | 44 | 74x |
| Nr. 2 (S.H.) | Lymphocyten (Gpt/l) | 1,5 - 3,5 | 1,12 | 1,86x | 2,12x | 1,34 | 1,24 | |
| CDC: IVb | CD 3 (Gpt/l) | 1,0 - 2,0 | 0,01 | 0,02 | 0,53 | 0,43 | 0,71 | |
| | CD 4 (Gpt/l) | 0,6 - 1,3 | 0,06 | 0,13 | 0,15 | 0,11 | 0,17 | |
| | CD 4 / CD 8 | 1,5 - 3,0 | 0,18 | 0,20 | 0,22 | 0,22 | 0,24 | |
| | $DR^+$-Monocyten (Gpt/l) | 0 - 0,6 | 0,08 | 0,16 | n.b. | 0,20 | 0,32 | |
| | $DR^+$-Monocyten (%) | 65 - 90 | 38 | 30 | n.b. | 40 | 37 | |

2) die mit (x) gekennzeichneten Werte entsprechen Normalwerten
3) n.b. = nicht bestimmt

Tabelle 6.1.b Cytofluorometrischer Nachweis (quantitative Bestimmung) von Monocyten bzw. Lymphocytensubpopulationen mittels monoklonaler Antikörper bei HIV-infizierten Patienten vor und unter Therapie mit Diacétyl-Splenopentin

| Patienten (Krankheitsstadien) | Parameter | Normalwerte | Vor Behandlung | Therapie mit Splenopentin (Wochen) 4 | 8 | 12 | 16 | 24 |
|---|---|---|---|---|---|---|---|---|
| Nr. 3 (B.A.) | Lymphocyten (Gpt/l) | 1,5 - 3,5 | 1,3 | 3,30$^x$ | 3,81$^x$ | 2,53$^x$ | 1,84$^x$ [2)] | |
| CDC: IIIb | CD 3 (Gpt/l) | 1,0 - 2,0 | 0,05 | 0,53 | 1,18$^x$ | 1,78$^x$ | 1,20$^x$ | |
| | CD 4 (Gpt/l) | 0,6 - 1,3 | 0,02 | n.b.[3)] | 0,34 | 0,30 | 0,93 | |
| | CD 4 / CD 8 | 1,5 - 3,0 | 0,05 | n.b. | 0,21 | 0,27 | 0,72 | |
| | DR$^+$-Monocyten (Gpt/l) | 0 - 0,6 | 0,03 | 0,13 | n.b. | 0,24 | 0,41 | |
| | DR$^+$-Monocyten (%) | 65 - 90 | 5 | 32 | n.b. | 54 | 67$^x$ | |
| Nr. 4 (S.F.) | Lymphozyten (Gpt/l) | 1,5 - 3,5 | 1,30 | 2,02$^x$ | 1,07 | n.b. | 2,24$^x$ | |
| CDC: IIb | CD 3 (Gpt/l) | 1,0 - 2,0 | 0,26 | 0,68 | 0,75 | n.b. | 0,74 | |
| | CD 4 (Gpt/l) | 0,6 - 1,3 | 0,08 | 0,04 | 0,47 | n.b. | 0,81$^x$ | |
| | CD 4 / CD 8 | 1,5 - 3,0 | 0,06 | 0,20 | 0,27 | n.b. | 1,10$^x$ | |
| | DR$^+$-Monocyten (Gpt/l) | 0 - 0,6 | 0,11 | 0,23 | 0,22 | n.b. | 0,45 | |
| | DR$^+$-Monocyten (%) | 65 - 90 | 18 | 52 | 53 | n.b. | 72$^x$ | |

2) die mit (x) gekennzeichneten Werte entsprechen Normalwerten

3) n.b. = nicht bestimmt

**Beispiel 17:** Verhinderung immunsuppressiver Effekte chronischer Intoxikationen

Tab. 7

| Wirkung chronischer Alkoholintoxikation auf ausgewählte Parameter des Immunsystems von männlichen Balb/c-Mäusen | | | | |
|---|---|---|---|---|
| Gabe von 15 % Ethanol über 1,5 Monate<br>0,1 mg DAc-SP5 i.p., 5x täglich vor Test | +<br>- (n = 40) | +<br>+ (n = 40) | -<br>+ (n = 40) | -<br>- (n = 40) |
| % phagocytierende Peritonealzellen | 29,0±1,7 | 47,5±1,7 | 75,0±2,6 | 46,0±1,7 |
| Phagocytoseindex | 3,0±0,08 | 4,5±0,1 | 4,9±0,15 | 3,7±0,08 |
| Thymusmasse (mg) | 15,0±2,6 | 29,0±2,2 | 40,0±3,0 | 25,0±2,3 |
| Milzmasse (mg) | 60,0±3,2 | 82,0±5,6 | 95,0±7,1 | 85,0±3,6 |
| Plaque-bildende Zellen der Milz pro $10^6$ Zellen (Jerne-Plaquetest) | 61,3±3,8 | 214,3±5,9 | 285,0±11,3 | 156,6±12,0 |
| Durchschnittswerte ± Streuung. | | | | |

**Beispiel 18:** Therapie von Autoimmunreaktionen

Tab. 8.1.: Normalisierender Effekt von DAc-SP5 auf die induzierte Autoimmunreaktion gegenüber neuronalen Antigenen beim Kaninchen

| Versuchsgruppen[1] | % Antigen-spezifische Reaktion von Basophilen mit Neuroantigen (Kaninchen)-Standardpräparation | | | |
|---|---|---|---|---|
| | Ausgangswerte | nach 2 Wo. | nach 6 Wo. | nach 8 Wo. |
| 1. Hippocampus-Läsion[2) + DAc-SP5[3) n[4) = 6 | 31,3 ± 1,9 | 42,0 ± 4,8 | 28,3 ± 3,9 | 15,0 ± 2,9 |
| | | | * | * |
| 2. Hippocampusläsion + NaCl n = 6 | 28,0 ± 4,0 | 44,0 ± 6,7 | 46,6 ± 7,0 | 38,6 ± 0,7 |
| 3. Kontrolle + DAc-SP5 n = 4 | 21,0 ± 5,0 | 20,5 ± 3,3 | 10,0 ± 2,7 | 23,0 ± 6,2 |
| 4. Kontrolle + NaCl n = 4 | 11,0 ± 3,0 | 20,5 ± 4,6 | 33,5 ± 3,3 | 22,5 ± 3,8 |

1) Chinchilla-Kaninchen (ca. 3000g) mit spontaner Autoimmun-Reaktion gegen neuronale Antigene.

2) Lokale Läsion des dorsalen Hippocampus führt zu einer Erhöhung der Autoimmunreaktion gegen neuronale Antigene.

3) Gabe von DAc-SP5 (1mg/kg, s.c.) 5 x im Abstand von 4 Tagen.

* ≙ $p < 0{,}005$ (t-Test nach Student)

4) n = Anzahl der Tiere

Tabl. 8.2.: Wirkung von DAc-SP5 auf den Titer von (Anti-Ovalbumin)-Antikörpern[1)]

| $\log_2$ PCA-Titer | Gruppe I | Gruppe II | Gruppe III | Gruppe IV |
|---|---|---|---|---|
| Immunisierung | | | | |
| 2. Booster | 9,3 | 10,3 | 10,3 | 9,3 |
| 3. Booster | 12,3 | 12,3 | 13,3 | 12,3 |
| 4. Booster | 13,3 | 13,3 | 14,3 | 14,3 |

| | DAc-SP5 (3 x pro Woche, s.c.) | | | Kontrolle |
|---|---|---|---|---|
| Tage nach letzter Boosterung | Gruppe I[2)] 10 µg/kg | Gruppe II[2)] 100 µg/kg | Gruppe III[2)] 500 µg/kg | Gruppe IV |
| 7 | 13,6 ± 0,6 | 13,5 ± 0,7 | 13,6 ± 0,6 | 13,4 ± 0,5 |
| 14 | 13,5 ± 1,0 | 13,8 ± 0,8 | 13,8 ± 0,8 | 13,0 ± 0,8 |
| 21 | 13,0 ± 0,4 | 12,6 ± 0,4 | 12,8 ± 0,5 | 12,7 ± 0,5 |
| 28 | 12,9 ± 1,3 | 12,9 ± 0,8 | 13,0 ± 1,0 | 13,0 ± 0,9 |
| 35 | 12,8 ± 0,5 | 13,2 ± 0,5 | 12,7 ± 0,5 | 12,9 ± 0,5 |
| 42 | 13,0 ± 0,6 | 13,1 ± 0,9 | 12,7 ± 0,5 | 12,9 ± 0,5 |
| 49 | 12,9 ± 0,7 | 13,1 ± 0,9 | 12,6 ± 0,6 | 12,6 ± 0,4 |
| 56 | 12,3 ± 0,4 | 12,6 ± 0,9 | 12,1 ± 0,9 | 12,1 ± 0,4 |
| | $\bar{x}$ ± S.D. n = 10 | $\bar{x}$ ± S.D. n = 10 | $\bar{x}$ ± S.D. n = 10 | $\bar{x}$ ± S.D. n = 10 |

[1)] Es wurden $F_1$-Hybridmäuse (Balb/c x C57 BL/6J) mit 1 µg Ovalbumin (emulgiert in 1 mg $Al(OH)_3$) immunisiert.
Der Anti-Ovalbumin-Titer wurde mit Hilfe der Methode der passiven kutanen Anaphylaxie (Wyczotkowska et al., Int. Archs. Allergy appl. Immun. 72 (1983) 16-21 anhand von Verdünnungsreihen des Serums der Einzeltiere bestimmt.

[2)] Die Werte von Versuchs- und Kontrollgruppe weisen keinen statistisch signifikanten Unterschied auf.

**Patentansprüche**
**Patentansprüche für folgende Vertragsstaaten : AT, BE, CH, DE, FR, GB, IT, LI, NL, SE**

1. Modifizierte Splenopentine der Formel I

   $R^1$-Arg-Lys($R^2$)-Glu-Val-Tyr($R^3$)$R^4$ (I),

   worin bedeuten:
   $R^1$: Wasserstoff, $C_{1-7}$-Alkyl, $C_{5-12}$-Aryl, $C_{6-20}$-Alkaryl, $C_{6-20}$-Alalkyl, $C_{1-8}$-Alkanoyl, $C_{1-7}$-Alkenyl, $C_{1-7}$-Alkinyl, $C_{6-12}$-Polyhydroxyalkanoyl, -CO($CH_2$)$_n$-COOH (n = 1-8), -CO(CHOH)$_n$-COOH (n = 1-12), -CO-($CH_2$)$_n$-CO-Arg-Lys-Glu-Val-Tyr (n = 1-8) oder -CO-(CHOH)$_n$-CO-Arg-Lys-Glu-Val-Tyr (n = 1-12);
   $R^2$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl, $C_{1-7}$-Alkinyl, $C_{6-12}$-Polyhydroxyalkanoyl, -CO($CH_2$)$_n$-COOH (n = 1-8), -CO(CHOH)$_n$-COOH (n = 1-12, -CO($CH_2$)$_n$-CO-($N^\epsilon$-Lys)-splenopentin (n = 1-8) oder -CO(CHOH)$_n$-CO-($N^\epsilon$-Lys)-splenopentin (n = 1-12);
   $R^3$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl oder $C_{1-7}$-Alkinyl
   und
   $R^4$: OH, $NH_2$, $NHR^5$, $NR^5{}_2$ oder $OR^5$, worin $R^5$ bedeutet: $C_{1-18}$-Alkyl, $C_{1-7}$-Alkenyl, $C_{1-7}$-Alkinyl, $C_{6-20}$-Aralkyl, $C_{6-20}$-Alkaryl, -(CHOH)$_n$-Polyhydroxyalkyl (n = 1-8), -($CH_2$)-NH-Tyr-Val-Glu-Lys-Arg (m = 1-18), -($CH_2CH_2$-O)$_m$-Tyr-Val-Glu-Lys-Arg (m = 1-10000) oder -($CH_2CH_2$-O)$_m$-NH-Tyr-Val-Glu-Lys-Arg (m = 1-10000),

   wobei Splenopentin ($R^1 = R^2 = R^3 = H$; $R^4$-OH) ausgenommen ist und $N^\alpha$-Arg-Substitution, $N^\epsilon$-Lys-Substitution oder $N^\alpha$-Arg- und $N^\epsilon$-Lys-Substitution vorliegt,

2. Folgende Verbindungen der Formel I nach Anspruch 1:
   $N^\alpha$-Arg,$N^\epsilon$-Lys,O-Tyr-Triacetylsplenopentin,
   $N^\alpha$-Arg,$N^\epsilon$-Lys-Diacetylsplenopentin (DAc-SP5),
   $N^\alpha$-Arg-Acetylsplenopentin ($N^\alpha$-MAc-SP5),
   $N^\epsilon$-Lys-Acetylsplenopentin ($N^\epsilon$-MAc-SP5),
   D-Tyr-$N^\alpha$-Arg,$N^\epsilon$-Lys-Diacetylsplenopentin,
   L-Tyr-$N^\alpha$-Arg,$N^\epsilon$-Lys-Diacetylsplenopentin,
   $N^\epsilon$-Lys-Palmitoylsplenopentin ($N^\epsilon$-Pal-SP5) oder
   $N^\alpha$-Arg-Palmitoylsplenopentin ($N^\alpha$-Pal-SP5)

3. Verfahren zur Herstellung acylierter Splenopentine der Formel I nach Anspruch 1 oder 2, gekennzeichnet durch Acylierung von Splenopentin sowie partiell geschützten Splenopentinen.

4. Verfahren nach Anspruch 3, dadurch gekennzeichnet, daß die Acylierung des Splenopentins bzw. der partiell geschützten Splenopentine mit N-Hydroxynorborn-5-en-2,3-dicarboximidestern der entsprechenden Carbonsäuren, vorzugsweise N-Acetoxynorborn-5-en-2,3-dicarboximid (Acetyl-ONB), durchgeführt wird.

5. Verfahren nach Anspruch 3, dadurch gekennzeichnet, daß die Acylierung des Splenopentins bzw. der partiell geschützten Splenopentine mit 1-Acetoxybenzotriazol (Acetyl-OBt) durchgeführt wird.

6. Verfahren nach einem der Ansprüche 3 bis 5 zur Herstellung von ($N^\alpha$-Acetyl-arginyl)($N^\epsilon$-acetyl-lysyl)-glutamylvalyltyrosin (DAc-SP5), gekennzeichnet durch Umsetzung von Splenopentin Arg-Lys-Glu-Val-Tyr mit N-Acetoxynorborn-5-en-2,3-dicarboximid bzw. 1-Acetoxybenzotriazol.

7. Verfahren nach einem der Ansprüche 3 bis 5 zur Herstellung von Arginyl($N^\epsilon$-acetyl-lysyl)-glutamylvalyltyrosin ($N^\epsilon$-MAc-SP5), gekennzeichnet durch Umsetzung von $N^\epsilon$-Arggeschütztem Splenopentin mit N-Acetoxynorborn-5-en-2,3-dicarboximid bzw. 1-Acetoxybenzotriazol und nachfolgende Abspaltung der $N^\alpha$-Schutzgruppe.

8. Verfahren nach einem der Ansprüche 3 bis 7, dadurch gekennzeichnet, daß die acylierten Splenopentine an carboxylierten Kationenaustauschern, vorzugsweise Kationenaustauschern auf der Basis von mit

Divinylbenzol vernetzten Acrylaten, in wäßrigen Elutionssystemen bei Ionenstärken zwischen $10^{-5}$ und $10^{-2}$ mol/l entsprechend ihrer Polarität getrennt werden, wobei die Elution vorzugsweise isokratisch mit 10 bis 20 Masse-% Ethanolanteil im wäßrigen Elutionsmittel bei pH 2,5 durchgeführt wird.

**9.** Pharmazeutische Mittel, gekennzeichnet durch mindestens eine Verbindung der Formel I nach Anspruch 1 oder 2 und einen oder mehrere pharmakologisch geeignete Hilfs- und/oder Träerstoffe und/oder ein physiologisch verträgliches Verdünnungsmittel.

**10.** Verwendung der modifizierten Splenopentine der Formel I nach Anspruch 1 oder 2 zur Herstellung von Arzneimitteln zur Normalisierung des Immunsystems, insbesondere zur Behandlung viraler Infektionen, zur Behandlung nach chemotherapeutischen Maßnahmen, zur Stimulierung des Wachstums und der Reifung von Knochenmarkzellen, zur Therapie von HIV-Infektionen, zur Verhinderung immunsuppressiver Wirkungen bei chronischen Intoxikationen, wie chronischem Alkoholkonsum, sowie zur Therapie von Autoimmunreaktionen.

**Patentansprüche für folgenden Vertragsstaat : ES**

**1.** Verfahren zur Herstellung modifizierter Splenopentine der Formel I,

$R^1$-Arg-Lys($R^2$)-Glu-Val-Tyr($R^3$)$R^4$ (I),

worin bedeuten:

$R^1$: Wasserstoff, $C_{1-7}$-Alkyl, $C_{5-12}$-Aryl, $C_{6-20}$-Alkaryl, $C_{6-20}$-Aralkyl, $C_{1-18}$-Alkanoyl, $C_{1-7}$-Alkenyl, $C_{1-7}$-Alkinyl, $C_{6-12}$Polyhydroxyalkanoyl, -CO($CH_2$)$_n$-COOH (n = 1-8), -CO(CHOH)$_n$-COOH (n = 1-12), -CO-($CH_2$)$_n$-CO-Arg-Lys-Glu-Val-Tyr (n = 1-8) oder -CO-(CHOH)$_n$-Co-Arg-Lys-Glu-Val-Tyr (n = 1-12);

$R^2$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl, $C_{1-7}$-Alkinyl, $C_{6-12}$-Polyhydroxyalkanoyl, -CO($CH_2$)$_n$-COOH (n = 1-8), -CO(CHOH)$_n$-COOH (n = 1-12, -CO($CH_2$)$_n$-CO-($N^\epsilon$-Lys)-splenopentin (n = 1-8) oder -CO(CHOH)$_n$-CO-($N^\epsilon$-Lys)-splenopentin (n = 1-12);

$R^3$: Wasserstoff, $C_{1-7}$-Alkyl, $C_{5-12}$-Aryl, $c_{6-20}$-Alkaryl, $C_{1-18}$-Alkanoyl, $C_{1-7}$-Alkenyl oder $C_{1-7}$-Alkinyl

und

$R^4$: OH, $NH_2$, $NHR^5$, $NR^5{}_2$ oder $OR^5$, worin $R^5$ bedeutet: $C_{1-18}$-Alkyl, $C_{1-7}$-Alkenyl, $C_{1-7}$-Alkinyl, $C_{6-20}$-Aralkyl, $C_{6-20}$-Alkaryl, -(CHOH)$_n$-Polyhydroxyalkyl (n = 1-8), -($CH_2$)-NH-Tyr-Val-Glu-Lys-Arg (m = 1-18), -($CH_2CH_2$-O)$_m$-Tyr-Val-Glu-Lys-Arg (m = 1-10000) oder -($CH_2CH_2$-O)$_m$-NH-Tyr-Val-Glu-Lys-Arg (m = 1-10000),

wobei Splenopentin ($R^1 = R^2 = R^3 = H$; $R^4 = OH$) ausgenommen ist und $N^\alpha$-Arg-Substitution, $N^\epsilon$-Lys-Substitution oder $N^\alpha$-Arg- und $N^\epsilon$-Lys-Substitution vorliegt,
gekennzeichnet durch
Acylierung von Splenopentin sowie entsprechender partiell geschützter Splenopentine.

**2.** Verfahren nach Anspruch 1, wobei
$N^\alpha$-Arg,$N^\epsilon$-Lys,O-Tyr-Triacetylsplenopentin,
$N^\alpha$-Arg,$N^\epsilon$-Lys-Diacetylsplenopentin (DAc-SP5),
$N^\alpha$-Arg-Acetylsplenopentin ($N^\alpha$-MAc-SP5),
$N^\epsilon$-Lys-Acetylsplenopentin ($N^\epsilon$-MAc-SP5),
D-Tyr-$N^\alpha$-Arg,$N^\epsilon$-Lys-Diacetylsplenopentin,
L-Tyr-$N^\alpha$-Arg,$N^\epsilon$-Lys-Diacetylsplenopentin,
$N^\epsilon$-Lys-Palmitoylsplenopentin ($N^\epsilon$-Pal-SP5) oder
$N^\alpha$-Arg-Palmitoylsplenopentin ($N^\alpha$-Pal-SP5)
hergestellt werden.

**3.** Verfahren nach Anspruch 1 oder 2, dadurch gekennzeichnet, daß die Acylierung des Splenopentins bzw. der partiell geschützten Splenopentine mit N-Hydroxynorborn-5-en-2,3-dicarboximidestern der entsprechenden Carbonsäuren, vorzugsweise N-Acetoxynorborn-5-en-2,3-dicarboximid (Acetyl-ONB), durchgeführt wird.

4. Verfahren nach Anspruch 1 oder 2, dadurch gekennzeichnet, daß die Acylierung des Splenopentins bzw. der partiell geschützten Splenopentine mit 1-Acetoxybenzotriazol (Acetyl-OBt) durchgeführt wird.

5. Verfahren nach einem der Ansprüche 1 bis 4 zur Herstellung von ($N^{\alpha}$-Acetyl-arginyl)($N^{\epsilon}$-acetyl-lysyl)-glutamylvalyltyrosin (DAc-SP5), gekennzeichnet durch Umsetzung von Splenopentin Arg-Lys-Glu-Val-Tyr mit N-Acetoxynorborn-5-en-2,3-dicarboximid bzw. 1-Acetoxybenzotriazol.

6. Verfahren nach einem der Ansprüche 1 bis 4 zur Herstellung von Arginyl($N^{\epsilon}$-acetyl-lysyl)-glutamylvalyltyrosin ($N^{\epsilon}$-MAc-SP5), gekennzeichnet durch Umsetzung von $N^{\epsilon}$-Arggeschütztem Splenopentin mit N-Acetoxynorborn-5-en-2,3-dicarboximid bzw. 1-Acetoxybenzotriazol und nachfolgende Abspaltung der $N^{\alpha}$-Schutzgruppe.

7. Verfahren nach einem der Ansprüche 1 bis 6, dadurch gekennzeichnet, daß die acylierten Splenopentine an carboxylierten Kationenaustauschern, vorzugsweise Kationenaustauschern auf der Basis von mit Divinylbenzol vernetzten Acrylaten, in wäßrigen Elutionssystemen bei Ionenstärken zwischen $10^{-5}$ und $10^{-2}$ mol/l entsprechend ihrer Polarität getrennt werden, wobei die Elution vorzugsweise isokratisch mit 10 bis 20 Masse-% Ethanolanteil im wäßrigen Elutionsmittel bei pH 2,5 durchgeführt wird.

8. Verfahren zur Herstellung pharmazeutischer Mittel auf der Basis modifizierter Splenopentine, gekennzeichnet durch
Vermischen von einem oder mehreren modifizierten Splenopentinen der Formel I,

$R^1$-Arg-Lys($R^2$)-Glu-Val-Tyr($R^3$)$R^4$ (I),

worin bedeuten:

$R^1$: Wasserstoff, $C_{1-7}$-Alkyl, $C_{5-12}$-Aryl, $C_{6-20}$-Alkaryl, $C_{6-20}$-Aralkyl, $C_{1-18}$-Alkanoyl, $C_{1-7}$-Alkenyl, $C_{1-7}$-Alkinyl, $C_{6-12}$-Polyhydroxyalkanoyl, $-CO(CH_2)_n-COOH$ (n = 1-8), $-CO(CHOH)_n-COOH$ (n = 1-12), $-CO-(CH_2)_n$-CO-Arg-Lys-Glu-Val-Tyr (n = 1-8) oder $-CO-(CHOH)_n$-CO-Arg-Lys-Glu-Val-Tyr (n = 1-12);

$R^2$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl, $C_{1-7}$-Alkinyl, $C_{6-12}$-Polyhydroxyalkanoyl, $-CO(CH_2)_n-COOH$ (n = 1-8), $-CO(CHOH)_n-COOH$ (n = 1-12, $-CO(CH_2)_n-CO-(N^{\epsilon}$-Lys)-splenopentin (n = 1-8) oder $-CO(CHOH)_n-CO-(N^{\epsilon}$-Lys)-splenopentin (n = 1-12);

$R^3$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl oder $C_{1-7}$-Alkinyl

und

$R^4$: OH, $NH_2$, $NHR^5$, $NR^5{}_2$ oder $OR^5$, worin $R^5$ bedeutet: $C_{1-18}$-Alkyl, $C_{1-7}$-Alkenyl, $C_{1-7}$-Alkinyl, $C_{6-20}$-Aralkyl, $C_{6-20}$-Alkaryl, $-(CHOH)_n$-Polyhydroxyalkyl (n = 1-8), $-(CH_2)_m$-NH-Tyr-Val-Glu-Lys-Arg (m = 1-18), $-(CH_2CH_2-O)_m$-Tyr-Val-Glu-Lys-Arg (m = 1-10000) oder $-(CH_2CH_2-O)_m$-NH-Tyr-Val-Glu-Lys-Arg (m = 1-10000),

wobei Splenopentin ($R^1 = R^2 = R^3 = H$; $R^4 = OH$) ausgenommen ist und $N^{\alpha}$-Arg-substitution, $N^{\epsilon}$-Lys-Substitution oder $N^{\alpha}$-Arg- und $N^{\epsilon}$-Lys-Substitution vorliegt,
mit einem oder mehreren pharmazeutisch akzeptablen Hilfs- und/oder Trägerstoffen und/oder physiologisch verträglichen Verdünnungsmitteln.

9. Verwendung modifizierter Splenopentine der Formel I,

$R^1$-Arg-Lys($R^2$)-Glu-Val-Tyr($R^3$)$R^4$ (I),

worin bedeuten:

$R^1$: Wasserstoff, $C_{1-7}$-Alkyl, $C_{5-12}$-Aryl, $C_{6-20}$-Alkaryl, $C_{6-20}$-Aralkyl, $C_{1-18}$-Alkanoyl, $C_{1-7}$-Alkenyl, $C_{1-7}$-Alkinyl, $C_{6-12}$-Polyhydroxyalkanoyl, $-CO(CH_2)_n-COOH$ (n = 1-8), $-CO(CHOH)_n-COOH$ (n = 1-12), $-CO-(CH_2)_n$-CO-Arg-Lys-Glu-Val-Tyr (n = 1-8) oder $-CO-(CHOH)_n$-CO-Arg-Lys-Glu-Val-Tyr (n = 1-12);

$R^2$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl, $C_{1-7}$-Alkinyl, $C_{6-12}$-Polyhydroxyalkanoyl, $-CO(CH_2)_n-COOH$ (n = 1-8), $-CO(CHOH)_n-COOH$ (n = 1-12, $-CO(CH_2)_n-CO-(N^{\epsilon}$-Lys)-splenopentin (n = 1-8) oder $-CO(CHOH)_n-CO-(N^{\epsilon}$-Lys)-splenopentin (n = 1-12);

$R^3$: Wasserstoff, $C_{1-7}$-Alkyl, $C_{5-12}$-Aryl, $C_{6-20}$-Alkaryl, $C_{1-18}$-Alkanoyl, $C_{1-7}$-Alkenyl oder $C_{1-7}$-Alkinyl

und

$R^4$: OH, $NH_2$, $NHR^5$, $NR^5{}_2$ oder $OR^5$, worin $R^5$ bedeutet: $C_{1-18}$-Alkyl, $C_{1-7}$-Alkenyl, $C_{1-7}$-Alkinyl, $C_{6-20}$-Aralkyl, $C_{6-20}$-Alkaryl, $-(CHOH)_n$-Polyhydroxyalkyl (n = 1-8), $-(CH_2)_m$-NH-Tyr-Val-Glu-Lys-Arg (m = 1-18), $-(CH_2CH_2-O)_m$-Tyr-Val-Glu-Lys-Arg (m = 1-10000) oder $-(CH_2CH_2-O)_m$-NH-Tyr-Val-Glu-Lys-Arg (m = 1-10000),

wobei Splenopentin ($R^1 = R^2 = R^3 = H$; $R^4 = OH$) ausgenommen ist und $N^\alpha$-Arg-Substitution, $N^\epsilon$-Lys-Substitution oder $N^\alpha$-Arg- und $N^\epsilon$-Lys-Substitution vorliegt,

zur Herstellung von Arzneimitteln zur Normalisierung des Immunsystems, insbesondere zur Behandlung viraler Infektionen, zur Behandlung nach chemotherapeutischen Maßnahmen, zur Stimulierung des Wachstums und der Reifung von Knochenmarkzellen, zur Therapie von HIV-Infektionen, zur Verhinderung immunsuppressiver Wirkungen bei chronischen Intoxikationen, wie chronischem Alkoholkonsum, sowie zur Therapie von Autoimmunreaktionen.

**Claims**

**Claims for the following Contracting States : AT, BE, CH, DE, FR, GB, IT, LI, NL, SE**

**1.** Modified splenopentines having the formula I

$R^1$-Arg-Lys($R^2$)-Glu-Val-Tyr($R^3$)$R^4$ (I)

in which:

$R^1$ denotes hydrogen, $C_{1-7}$-alkyl, $C_{5-12}$-aryl, $C_{6-20}$-alkaryl, $C_6$-$_{20}$-aralkyl, $C_{1-18}$-alkanoyl, $C_{1-7}$alkenyl, $C_{1-7}$-alkinyl, $C_{6-12}$-polyhydroxyalkanoyl,
$-CO(CH_2)_n-COOH$ (n = 1-8),
$-CO(CHOH)_n-COOH$ (n = 1-12),
$-CO-(CH_2)_n$-CO-Arg-Lys-Glu-Val-Tyr (n = 1-8) or
$-CO-(CHOH)_n$-CO-Arg-Lys-Glu-Val-Tyr (n = 1-12);

$R^2$ denotes hydrogen, $C_{1-7}$-alkyl, $C_{5-12}$-aryl, $C_{6-20}$-alkaryl, $C_{1-18}$-alkanoyl, $C_{1-7}$-alkenyl, $C_{1-7}$-alkinyl, $C_{6-12}$-polyhydroxyalkanoyl,
$-CO(CH_2)_n-COOH$ (n = 1-8),
$-CO(CHOH)_n-COOH$ (n = 1-12),
$-CO(CH_2)_n$-CO-($N^\epsilon$-Lys)-splenopentine (n = 1-8) or
$-CO(CHOH)_n$-CO-($N^\epsilon$-Lys)-splenopentine (n = 1-12);

$R^3$ denotes hydrogen, $C_{1-7}$-alkyl, $C_{5-12}$-aryl, $C_{6-20}$-alkaryl, $C_{1-18}$-alkanoyl, $C_{1-7}$-alkenyl or $C_{1-7}$-alkinyl and

$R^4$ denotes OH, $NH_2$, $NHR^5$, $NR^5_2$ or $OR^5$, where $R^5$ denotes:
$C_{1-18}$-alkyl, $C_{1-7}$-alkenyl, $C_{1-7}$-alkinyl, $C_{6-20}$-aralkyl, $C_{6-20}$-alkaryl,
$-(CHOH)_n$-polyhydroxyalkyl (n = 1-8),
$-(CH_2)_m$-NH-Tyr-Val-Glu-Lys-Arg (m = 1-18),
$-(CH_5CH_2-O)_m$-Tyr-Val-Glu-Lys-Arg (m = 1-10000) or
$-(CH_2CH_2-O)_m$-NH-Tyr-Val-Glu-Lys-Arg (m = 1-10000),

splenopentine ($R^1$ = $R^2$ = $R^3$ = H; $R^4$ = OH) being excluded and $N^\alpha$-Arg substitution, $N^\epsilon$-Lys substitution or $N^\alpha$-Arg and $N^\epsilon$-Lys substitution occurring.

**2.** The following compounds of formula I according to claim 1:
$N^\alpha$-Arg, $N^\epsilon$-Lys, O-Tyr-triacetyl splenopentine,
$N^\alpha$-Arg, $N^\epsilon$-Lys-diacetyl splenopentine (DAc-SP5),
$N^\alpha$-Arg-acetyl splenopentine ($N^\alpha$-MAc-SP5),
$N^\epsilon$-Lys-acetyl splenopentine ($N^\epsilon$-MAc-SP5),
D-Tyr-$N^\alpha$-Arg, $N^\epsilon$-Lys-diacetyl splenopentine,
L-Tyr-$N^\alpha$-Arg, $N^\epsilon$-Lys-diacetyl splenopentine,
$N^\epsilon$-Lys-palmitoyl splenopentine ($N^\epsilon$-Pal-SP5) and
$N^\alpha$-Arg-palmitoyl splenopentine ($N^\alpha$-Pal-SP5).

**3.** A process for preparation of acylated splenopentines of formula I according to claim 1 or 2, characterised by acylation of splenopentine and of partially protected splenopentines.

4. The process according to claim 3, characterised in that the acylation of splenopentine or of the partially protected splenopentines is effected with N-hydroxy norborn-5-ene-2,3-dicarboximide esters of the corresponding carboxylic acids, preferably N-acetoxy norborn-5-ene-2,3-dicarboximide (acetyl-ONB).

5. The process according to claim 3, characterised in that the acylation of the splenopentine or of the partially protected splenopentines is effected with 1-acetoxybenzotriazole (acetyl-OBt).

6. The process according to any of claims 3 to 5 for preparation of ($N^{\alpha}$-acetyl-arginyl)($N^{\epsilon}$-acetyl-lysyl)-glutamylvalyltyrosine (DAc-SP5), characterised by reaction of splenopentine Arg-Lys-Glu-Val-Tyr with N-acetoxynorborn-5-ene-2,3-dicarboximide or 1-acetoxybenzotriazole.

7. The process according to any of claims 3 to 5 for preparation of arginyl (N($N^{\epsilon}$-acetyl-lysyl)-glutamylvalyltyrosine ($N^{\epsilon}$-MAc-SP5), characterised by reaction of $N^{\epsilon}$-Arg-protected splenopentine with N-acetoxynorborn-5-ene-2,3-dicarboximide or 1-acetoxybenzotriazole and subsequent splitting off of the $N^{\alpha}$ protective group.

8. The process according to any of claims 3 to 7, characterised in that the acylated splenopentines are separated on carboxylated cation exchangers, preferably cation exchangers based on acrylates cross-linked with divinyl benzene, in aqueous elution systems at ionic strengths between $10^{-5}$ and $10^{-2}$ mol/l depending on their polarity, elution preferably being brought about isocratically with 10 to 20% by weight of ethanol in an aqueous eluent at pH 2.5.

9. A pharmaceutical composition, characterised by at least one formula I compound according to claim 1 or 2 and one or more pharmacologically suitable adjuvants and/or excipients and/or a physiologically compatible diluent.

10. Use of the modified splenopentines of formula I according to claim 1 or 2 for the preparation of drugs for normalising the immune system, more particularly for treatment of viral infections, for treatment after chemotherapeutic measures, for stimulating growth and maturation of bone-marrow cells, treatment of HIV infections, prevention ofimmune-suppressive effects in chronic intoxications such as chronic alcoholism, and treatment of autoimmune reactions.

**Claims for the following Contracting State : ES**

1. A process for the preparation of modified splenopentines having the formula I,

$R^1$-Arg-Lys($R^2$)-Glu-Val-Tyr($R^3$)$R^4$ (I),

in which:
$R^1$ denotes hydrogen, $C_{1-7}$-alkyl, $C_{5-12}$-aryl, $C_{6-20}$-alkaryl, $C_{6-20}$-aralkyl, $C_{1-18}$alkanoyl, $C_{1-7}$-alkenyl, $C_{1-7}$-alkinyl, $C_{6-12}$-polyhydroxyalkanoyl,
$-CO(CH_2)_n-COOH$ (n = 1-8),
$-CO(CHOH)_n-COOH$ (n = 1-12),
$-CO-(CH_2)_n-CO$-Arg-Lys-Glu-Val-Tyr (n = 1-8) or
$-CO-(CHOH)_n-CO$-Arg-Lys-Glu-Val-Tyr (n=1-12);
$R^2$ denotes hydrogen, $C_{1-7}$-alkyl, $C_{5-12}$-aryl, $C_{6-20}$-alkaryl, $C_{1-18}$-alkanoyl, $C_{1-7}$-alkenyl, $C_{1-7}$-alkinyl, $C_{6-12}$-polyhydroxyalkanoyl,
$-CO(CH_2)_n-COOH$ (n = 1-8),
$-CO(CHOH)_n-COOH$ (n = 1-12),
$-CO(CH_2)_n-CO-(N^{\epsilon}$-Lys)-splenopentine (n = 1-8) or
$-CO(CHOH)_n-CO-(N^{\epsilon}$-Lys)-splenopentine (n = 1-12);
$R^3$ denotes hydrogen, $C_{1-7}$-alkyl, $C_{5-12}$-aryl, $C_{6-20}$-alkaryl, $C_{1-18}$-alkanoyl, $C_{1-7}$-alkenyl or $C_{1-7}$-alkinyl, and
$R^4$ denotes OH, $NH_2$, $NHR^5$, $NR^5_2$ or $OR^5$, where $R^5$ denotes: $C_{1-18}$-alkyl, $C_{1-7}$-alkenyl, $C_{1-7}$-alkinyl, $C_{6-20}$-aralkyl, $C_{6-20}$-alkaryl,
$-(CHOH)_n$-polyhydroxyalkyl (n = 1-8),
$-(CH_2)_m$-NH-Tyr-Val-Glu-Lys-Arg (m = 1-18),
$-(CH_2CH_2-O)_m$-Tyr-Val-Glu-Lys-Arg (m = 1-10000) or

$-(CH_2CH_2-O)_m$-NH-Tyr-Val-Glu-Lys-Arg (m = 1-10000), splenopentine ($R^1$ = $R^2$ = $R^3$ = H; $R^4$ = OH) being excluded and $N^\alpha$-Arg substitution, $N^\epsilon$-Lys substitution or $N^\alpha$-Arg and $N^\epsilon$-Lys substitution occurring,
characterised by acylation of splenopentine and of corresponding partially protected splenopentines.

**2.** The process according to claim 1, wherein
$N^\alpha$-Arg, $N^\epsilon$-Lys, O-Tyr-triacetyl splenopentine,
$N^\alpha$-Arg, $N^\epsilon$-Lys-diacetyl splenopentine (DAc-SP5),
$N^\alpha$-Arg-acetyl splenopentine ($N^\alpha$-MAc-SP5),
$N^\epsilon$-Lys-acetyl splenopentine ($N^\epsilon$-MAc-SP5),
D-Tyr-$N^\alpha$-Arg, $N^\epsilon$-Lys-diacetyl splenopentine,
L-Tyr-$N^\alpha$-Arg, $N^\epsilon$-Lys-diacetyl spenopentine,
$N^\epsilon$-Lys-palmitoyl splenopentine ($N^\epsilon$-Pal-SP5)or
$N^\epsilon$-Arg-palmitoyl splenopentine ($N^\alpha$-Pal-SP5)
are prepared.

**3.** The process according to claim 1 or 2, characterised in that the acylation of the splenopentine or of the partially protected splenopentines is effected acylated with N-hydroxy norborn-5-ene-2,3-dicarboximide esters of the corresponding carboxylic acids, preferably N-acetoxy norborn-5-ene-2,3-dicarboximide (acetyl-ONB).

**4.** The process according to claim 1 or 2, characterised in that the acylation of the splenopentine or of the partially protected splenopentines is effected with 1-acetoxybenzotriazole (acetyl-OBt).

**5.** The process according to any of claims 1 to 4 for preparation of ($N^\alpha$-acetyl-arginyl)($N^\epsilon$-acetyl-lysyl)-glutamylvalyltyrosine (DAc-SP5), characterised by reaction of splenopentine Arg-Lys-Glu-Val-Tyr with N-acetoxynorborn-5-ene-2,3-dicarboximide or 1-acetoxybenzotriazole.

**6.** The process according to any of claims 1 to 4 for preparation of arginyl (N($N^\epsilon$-acetyl-lysyl)-glutamylvalyltyrosine ($N^\epsilon$-MAc-SP5), characterised by reaction of $N^\epsilon$-Arg-protected splenopentine with N-acetoxynorborn-5-ene-2,3-dicarboximide or 1-acetoxybenzotriazole and subsequent splitting off of the $N^\alpha$ protective group.

**7.** The process according to any of claims 1 to 6, characterised in that the acylated splenopentines are separated on carboxylated cation exchangers, preferably cation exchangers based on acrylates cross-linked with divinyl benzene, in aqueous elution systems at ionic strengths between $10^{-5}$ and $10^{-2}$ mol/l depending on their polarity, elution preferably being brought about isocratically with 10 to 20% by weight of ethanol in an aqueous eluent at pH 2.5.

**8.** A process for the preparation of pharmaceutical compositions on the basis of modified splenopentines, characterised by mixing one or more modified splenopentines having the formula I,

$R^1$-Arg-Lys($R^2$)-Glu-Val-Tyr($R^3$)$R^4$ (I),

in which:
$R^1$ denotes hydrogen, $C_{1-7}$-alkyl, $C_{5-12}$-aryl, $C_{6-20}$-alkaryl, $C_{6-20}$-aralkyl, $C_{1-18}$-alkanoyl, $C_{1-7}$-alkenyl, $C_{1-7}$-alkinyl, $C_{6-12}$polyhydroxyalkanoyl,
$-CO(CH_2)_n$-COOH (n = 1-8),
$-CO(CHOH)_n$-COOH (n = 1-12),
$-CO-(CH_2)_n$-CO-Arg-Lys-Glu-Val-Tyr (n = 1-8) or
$-CO-(CHOH)_n$-CO-Arg-Lys-Glu-Val-Tyr (n = 1-12);
$R^2$ denotes hydrogen, $C_{1-7}$-alkyl, $C_{5-12}$-aryl, $C_{6-20}$-alkaryl, $C_{1-18}$-alkanoyl, $C_{1-7}$-alkenyl, $C_{1-7}$-alkinyl, $C_{6-12}$-polyhydroxyalkanoyl,
$-CO(CH_2)_n$-COOH (n = 1-8),
$-CO(CHOH)_n$-COOH (n = 1-12),
$-CO(CH_2)_n$-CO-($N^\epsilon$-Lys)-splenopentine (n = 1-8) or
-CO(CHOH)n-CO-($N^\epsilon$-Lys)-splenopentine (n = 1-12);
$R^3$ denotes hydrogen, $C_{1-7}$-alkyl, $C_{5-12}$-aryl, $C_{6-20}$-alkaryl, $C_{1-18}$-alkanoyl, $C_{1-7}$-alkenyl or $C_{1-7}$-

alkinyl and
$R^4$ denotes OH, $NH_2$, $NHR^5$, $NR^5_2$ or $OR^5$, where $R^5$ denotes:
$C_{1-18}$-alkyl, $C_{1-7}$-alkenyl, $C_{1-7}$-alkinyl, $C_{6-20}$-aralkyl, $C_{6-20}$-alkaryl,
$-(CHOH)_n$-polyhydroxyalkyl (n = 1-8),
$-(CH_2)_m$-NH-Tyr-Val-Glu-Lys-Arg (m = 1-18),
$-(CH_2CH_2-O)_m$-Tyr-Val-Glu-Lys-Arg (m = 1-10000) or
$-(CH_2CH_2-O)_m$-NH-Tyr-Val-Glu-Lys-Arg (m = 1-10000),
splenopentine ($R^1$ = $R^2$ = $R^3$ = H; $R^4$ = OH) being excluded and $N^\alpha$-Argsubstitution, $N^\epsilon$-Lys substitution or $N^\alpha$-Arg and $N^\epsilon$-Lys substitution occurring,
with one or more pharmaceutically suitable adjuvants and/or excipients and/or physiologically compatible diluents.

**9.** Use of the modified splenopentines of formula I,

$R^1$-Arg-Lys($R^2$)-Glu-Val-Tyr($R^3$)$R^4$ (I),

in which:
$R^1$ denotes hydrogen, $C_{1-7}$-alkyl, $C_{5-12}$-aryl, $C_{6-20}$-alkaryl, $C_{6-20}$-aralkyl, $C_{1-18}$-alkanoyl, $C_{1-7}$-alkenyl, $C_{1-7}$-alkinyl, $C_{6-12}$-polyhydroxyalkanoyl,
$-CO(CH_2)_n$-COOH (n = 1-8),
$-CO(CHOH)_n$-COOH (n = 1-12),
$-CO-(CH_2)_n$-CO-Arg-Lys-Glu-Val-Tyr (n = 1-8) or
$-CO-(CHOH)_n$-CO-Arg-Lys-Glu-Val-Tyr (n=1-12);
$R^2$ denotes hydrogen, $C_{1-7}$-alkyl, $C_{5-12}$-aryl, $C_{6-20}$-alkaryl, $C_{1-18}$-alkanoyl, $C_{1-7}$-alkenyl, $C_{1-7}$-alkinyl, $C_{6-12}$-polyhydroxyalkanoyl,
$-CO(CH_2)_n$-COOH (n = 1-8),
$-CO(CHOH)_n$-COOH (n = 1-12),
$-CO(CH_2)_n$-CO-($N^\epsilon$-Lys)-splenopentine (n = 1-8) or
$-CO(CHOH)_n$-CO-($N^\epsilon$-Lys)-splenopentine (n = 1-12);
$R^3$ denotes hydrogen, $C_{1-7}$-alkyl, $C_{5-12}$-aryl, $C_{6-20}$-alkaryl, $C_{1-18}$-alkanoyl, $C_{1-7}$-alkenyl or $C_{1-7}$-alkinyl and
$R^4$ denotes OH, $NH_2$, $NHR^5$, $NR^5_2$ or $OR^5$, where $R^5$ denotes:
$C_{1-18}$-alkyl, $C_{1-7}$-alkenyl, $C_{1-7}$-alkinyl, $C_{6-20}$-aralkyl, $C_{6-20}$-alkaryl,
$-(CHOH)_n$-polyhydroxyalkyl (n = 1-8),
$-(CH_2)_m$-NH-Tyr-Val-Glu-Lys-Arg (m = 1-18),
$-(CH_2CH_2-O)_m$-Tyr-Val-Glu-Lys-Arg (m = 1-10000) or
$-(CH_2CH_2-O)_m$-NH-Tyr-Val-Glu-Lys-Arg (m = 1-10000),
splenopentine ($R^1$ = $R^2$ = $R^3$ = H; $R^4$ = OH) being excluded and $N^\alpha$-Arg substitution, $N^\epsilon$-Lys substitution or $N^\alpha$-Arg and $N^\epsilon$-Lys substitution occurring,
for the preparation of drugs for normalizing the immune system, more particularly for treatment of viral infections, for treatment after chemotherapeutic measures, stimulating growth and maturation of bone-marrow cells, treatment of HIV infections, prevention of immune-suppressive effects in chronic intoxications, such as chronic alcoholism, and treatment of autoimmune reactions.

## Revendications

### Revendications pour les Etats contractants suivants : AT, BE, CH, DE, FR, GB, IT, LI, NL, SE

**1.** Splénopentines modifiées de formule I

$R^1$-Arg-Lys($R^2$)-Glu-Val-Tyr($R^3$)$R^4$ (I),

dans laquelle :
$R^1$ représente l'hydrogène, un groupe alkyle en $C_{1-7}$, aryle en $C_{5-12}$, alkylaryle en $C_{6-20}$, aralkyle en $C_{6-20}$, alcanoyle en $C_{1-18}$, alcényle en $C_{1-7}$, alcynyle en $C_{1-7}$, polyhydroxyalcanoyle en $C_{6-12}$,
$-CO(CH_2)_n$-COOH (n = 1-8),
$-CO(CHOH)_n$-COOH (n = 1-12),
$-CO-(CH_2)_n$-CO-Arg-Lys-Glu-Val-Tyr (n = 1-8) ou

-CO-$(CHOH)_n$-CO-Arg-Lys-Glu-Val-Tyr (n = 1-12);

$R^2$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$, alcynyle en $C_{1-7}$, polyhydroxyalcanoyle en $C_{6-12}$, -CO$(CH_2)_n$-COOH (n = 1-8), -CO$(CHOH)_n$-COOH (n = 1-12), -CO$(CH_2)_n$-CO-($N^\epsilon$-Lys)-splénopentine (n = 1-8) ou -CO$(CHOH)_n$-CO-($N^\epsilon$-Lys)-splénopentine (n = 1-12);

$R^3$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$ ou alcynyle en $C_{1-7}$;

et

$R^4$ représente OH, $NH_2$, $NHR^5$, $NR^5_2$ , ou $OR^5$, où $R^5$ représente : un groupe alkyle en $C_{1-18}$, alcényle en $C_{1-7}$, alcynyle en $C_{1-7}$, aralkyle en $C_{6-20}$, alkylaryle en $C_{6-20}$, -$(CHOH)_n$-polyhydroxyalkyle (n = 1-8), -$(CH_2)_m$-NH-Tyr-Val-Glu-Lys-Arg (m = 1-18), -$(CH_2CH_2\text{-}O)_m$-Tyr-Val-Glu-Lys-Arg (m = 1-10000) ou -$(CH_2CH_2\text{-}O)_m$-NH-Tyr-Val-Glu-Lys-Arg (m = 1-10000),

à l'exception de la splénopentine ($R^1$ = $R^2$ = $R^3$ = H ; $R^4$ = OH) et en présence d'une substitution en $N^\alpha$-Arg, d'une substitution en $N^\epsilon$-Lys ou d'une substitution en $N^\alpha$-Arg et en $N^\epsilon$-Lys.

**2.** Composés suivants de formule I selon la revendication 1 :
$N^\alpha$-Arg, $N^\epsilon$-Lys, O-Tyr-triacétylsplénopentine,
$N^\alpha$-Arg, $N^\epsilon$-Lys-diacétylsplénopentine (DAc-SP5),
$N^\alpha$-Arg-acétylsplénopentine ($N^\alpha$-MAc-SP5),
$N^\epsilon$-Lys-acétylsplénopentine ($N^\epsilon$-MAc-SP5),
D-Tyr-$N^\alpha$-Arg, $N^\epsilon$-Lys-diacétylsplénopentine,
L-Tyr-$N^\alpha$-Arg, $N^\epsilon$-Lys-diacétylsplénopentine,
$N^\epsilon$-Lys-palmitoylsplénopentine ($N^\epsilon$-Pal-SP5) et
$N^\alpha$-Arg-palmitoylsplénopentine ($N^\alpha$-Pal-SP5).

**3.** Procédé de préparation de splénopentines acylées de formule I selon la revendication 1 ou 2, caractérisé par l'acylation de la splénopentine et de splénopentines partiellement protégées.

**4.** Procédé selon la revendication 3, caractérisé en ce que l'acylation de la splénopentine ou des splénopentines partiellement protégées est accomplie avec des esters de N-hydroxynorborn-5-ène-2,3-dicarboximide et des acides carboxyliques correspondants, de préférence avec le N-acétoxynorborn-5-ène-2,3-dicarboximide (acétyl-ONB).

**5.** Procédé selon la revendication 3, caractérisé en ce que l'acylation de la splénopentine ou des splénopentines partiellement protégées est accomplie avec le 1-acétoxybenzotriazole (acétyl-OBt).

**6.** Procédé selon l'une des revendications 3 à 5 pour la préparation de la ($N^\alpha$-acétyl-arginyl)($N^\epsilon$-acétyl-lysyl)-glutamylvalyltyrosine (DAc-SP5), caractérisé par la réaction de la splénopentine Arg-Lys-Glu-Val-Tyr avec le N-acétoxynorborn-5-ène-2,3-dicarboximide ou le 1-acétoxybenzotriazole.

**7.** Procédé selon l'une des revendications 3 à 5 pour la préparation de la ($N^\epsilon$-acétyl-lysyl)-glutamylvalyltyrosine ($N^\epsilon$-MAc-SP5), caractérisé par la réaction de la splénopentine $N^\epsilon$-Arg-protégée avec le N-acétoxynorborn-5-ène-2,3-dicarboximide ou le 1-acétoxybenzotriazole et la scission subséquente du groupe protecteur en $N^\alpha$.

**8.** Procédé selon l'une des revendications 3 à 7, caractérisé en ce que les splénopentines acylées sont séparées en fonction de leur polarité sur des échangeurs de cations carboxylés, de préférence des échangeurs de cations à base d'acrylates réticulés avec le divinylbenzène, dans des systèmes d'élution aqueux à des forces ioniques comprises entre $10^{-5}$ et $10^{-2}$ mol/l, l'élution étant réalisée de préférence de manière isocratique avec une proportion d'éthanol de 10 à 20 % en masse dans le milieu d'élution aqueux à pH 2,5.

**9.** Agents pharmaceutiques caractérisés par au moins un composé de formule I selon la revendication 1 ou 2 et un ou plusieurs adjuvants et/ou véhicules pharmacologiquement appropriés et/ou un diluant

physiologiquement acceptable.

**10.** Utilisation des splénopentines modifiées de formule I selon la revendication 1 ou 2 pour la préparation de médicaments pour la normalisation du système immunitaire, en particulier pour le traitement des infections virales, pour le traitement après des mesures chimiothérapeutiques, pour stimuler la croissance et la maturation des cellules médullaires, pour la thérapie des infections à HIV, pour empêcher des effets immunosuppresseurs dans les intoxications chroniques comme la consommation chronique d'alcool, et pour la thérapie des réactions auto-immunes.

**Revendications pour l'Etat contractant suivant : ES**

**1.** Procédé de préparation de
splénopentines modifiées de formule I,

$R^1$-Arg-Lys($R^2$)-Glu-Val-Tyr($R^3$)$R^4$ (I),

dans laquelle :

$R^1$ représente l'hydrogène, un groupe alkyle en $C_{1-7}$, aryle en $C_{5-12}$, alkylaryle en $C_{6-20}$, aralkyle en $C_{6-20}$, alcanoyle en $C_{1-18}$, alcényle en $C_{1-7}$, alcynyle en $C_{1-7}$, polyhydroxyalcanoyle en $C_{6-12}$,
-CO($CH_2$)$_n$-COOH (n = 1-8),
-CO(CHOH)$_n$-COOH (n = 1-12),
-CO-($CH_2$)$_n$-CO-Arg-Lys-Glu-Val-Tyr (n = 1-8) ou
-CO-(CHOH)$_n$-CO-Arg-Lys-Glu-Val-Tyr (n = 1-12);

$R^2$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$, alcynyle en $C_{1-7}$, polyhydroxyalcanoyle en $C_{6-12}$,
-CO($CH_2$)$_n$-COOH (n = 1-8),
-CO(CHOH)$_n$-COOH (n = 1-12),
-CO($CH_2$)$_n$-CO-($N^\epsilon$-Lys)-splénopentine (n = 1-8) ou
-CO(CHOH)$_n$-CO-($N^\epsilon$-Lys)-splénopentine (n = 1-12);

$R^3$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$ ou alcynyle en $C_{1-7}$;

et

$R^4$ représente OH, $NH_2$, $NHR^5$, $NR^5_2$ , ou $OR^5$, où $R^5$ représente:
un groupe alkyle en $C_{1-18}$, alcényle en $C_{1-7}$, alcynyle en $C_{1-7}$, aralkyle en $C_{6-20}$, alkylaryle en $C_{6-20}$,
-(CHOH)$_n$-polyhydroxyalkyle (n = 1-8),
-($CH_2$)$_m$-NH-Tyr-Val-Glu-Lys-Arg (m = 1-18),
-($CH_2CH_2$-O)$_m$-Tyr-Val-Glu-Lys-Arg (m = 1-10000) ou
-($CH_2CH_2$-O)$_m$-NH-Tyr-Val-Glu-Lys-Arg (m = 1-10000),

à l'exception de la splénopentine ($R^1$ = $R^2$ = $R^3$ = H ; $R^4$ = OH) et en présence d'une substitution en $N^\alpha$-Arg, d'une substitution en $N^\epsilon$-Lys ou d'une substitution en $N^\alpha$-Arg et en $N^\epsilon$-Lys,
caractérisé par l'acylation de la splénopentine et de splénopentines correspondantes partiellement protégées.

**2.** Procédé selon la revendication 1 pour préparer
$N^\alpha$-Arg, $N^\epsilon$-Lys, O-Tyr-triacétylsplénopentine,
$N^\alpha$-Arg, $N^\epsilon$-Lys-diacétylsplénopentine (DAc-SP5),
$N^\alpha$-Arg-acétylsplénopentine ($N^\alpha$-MAc-SP5),
$N^\epsilon$-Lys-acétylsplénopentine ($N^\epsilon$-MAc-SP5),
D-Tyr-$N^\alpha$-Arg, $N^\epsilon$-Lys-diacétylsplénopentine,
L-Tyr-$N^\alpha$-Arg, $N^\epsilon$-Lys-diacétylsplénopentine,
$N^\epsilon$-Lys-palmitoylsplénopentine ($N^\epsilon$-Pal-SP5) et
$N^\alpha$-Arg-palmitoylsplénopentine ($N^\alpha$-Pal-SP5).

**3.** Procédé selon la revendication 1 ou 2,
caractérisé en ce que l'acylation de la splénopentine ou des splénopentines partiellement protégées est accomplie avec des esters de N-hydroxynorborn-5-ène-2,3-dicarboximide et des acides carboxyliques

correspondants, de préférence avec le N-acétoxynorborn-5-ène-2,3-dicarboximide (acétyl-ONB).

**4.** Procédé selon la revendication 1 ou 2, caractérisé en ce que l'acylation de la splénopentine ou des splénopentines partiellement protégées est accomplie avec le 1-acétoxybenzotriazole (acétyl-OBt).

**5.** Procédé selon l'une des revendications 1 à 4 pour la préparation de la ($N^{\alpha}$-acétyl-arginyl)($N^{\epsilon}$-acétyl-lysyl)-glutamylvalyltyrosine (DAc-SP5), caractérisé par la réaction de la splénopentine Arg-Lys-Glu-Val-Tyr avec le N-acétoxynorborn-5-ène-2,3-dicarboximide ou le 1-acétoxybenzotriazole.

**6.** Procédé selon l'une des revendications 1 à 4 pour la préparation de la ($N^{\epsilon}$-acétyl-lysyl)-glutamylvalyltyrosine ($N^{\epsilon}$-MAc-SP5), caractérisé par la réaction de la splénopentine $N^{\epsilon}$-Arg-protégée avec le N-acétoxynorborn-5-ène-2,3-dicarboximide ou le 1-acétoxybenzotriazole et la scission subséquente du groupe protecteur en $N^{\alpha}$.

**7.** Procédé selon l'une des revendications 1 à 6, caractérisé en ce que les splénopentines acylées sont séparées en fonction de leur polarité sur des échangeurs de cations carboxylés, de préférence des échangeurs de cations à base d'acrylates réticulés avec le divinylbenzène, dans des systèmes d'élution aqueux à des forces ioniques comprises entre $10^{-5}$ et $10^{-2}$ mol/l, l'élution étant réalisée de préférence de manière isocratique avec une proportion d'éthanol de 10 à 20 % en masse dans le milieu d'élution aqueux à pH 2,5.

**8.** Procédé de préparation de compositions pharmaceutiques à la base de splénopentines modifiées, caractérisé en ce qu'on mélange un ou plusieurs splénopentines modifiées de formule I,

$R^1$-Arg-Lys($R^2$)-Glu-Val-Tyr($R^3$)$R^4$ (I),

dans laquelle :

$R^1$ représente l'hydrogène, un groupe alkyle en $C_{1-7}$, aryle en $C_{5-12}$, alkylaryle en $C_{6-20}$, aralkyle en $C_{6-20}$, alcanoyle en $C_{1-18}$, alcényle en $C_{1-7}$, alcynyle en $C_{1-7}$, polyhydroxyalcanoyle en $C_{6-12}$,
-$CO(CH_2)_n$-COOH (n = 1-8),
-$CO(CHOH)_n$-COOH (n = 1-12),
-CO-$(CH_2)_n$-CO-Arg-Lys-Glu-Val-Tyr (n = 1-8) ou
-CO-$(CHOH)_n$-CO-Arg-Lys-Glu-Val-Tyr (n = 1-12);

$R^2$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$, alcynyle en $C_{1-7}$, polyhydroxyalcanoyle en $C_{6-12}$,
-$CO(CH_2)_n$-COOH (n = 1-8),
-$CO(CHOH)_n$-COOH (n = 1-12),
-$CO(CH_2)_n$-CO-($N^{\epsilon}$-Lys)-splénopentine (n = 1-8) ou
-$CO(CHOH)_n$-CO-($N^{\epsilon}$-Lys)-splénopentine (n = 1-12);

$R^3$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$ ou alcynyle en $C_{1-7}$;

et

$R^4$ représente OH, $NH_2$, $NHR^5$, $NR^5_2$, ou $OR^5$, où $R^5$ représente:
un groupe alkyle en $C_{1-18}$, alcényle en $C_{1-7}$, alcynyle en $C_{1-7}$, aralkyle en $C_{6-20}$, alkylaryle en $C_{6-20}$,
-$(CHOH)_n$-polyhydroxyalkyle (n = 1-8),
-$(CH_2)_m$-NH-Tyr-Val-Glu-Lys-Arg (m = 1-18),
-$(CH_2CH_2\text{-}O)_m$-Tyr-Val-Glu-Lys-Arg (m = 1-10000) ou
-$(CH_2CH_2\text{-}O)_m$-NH-Tyr-Val-Glu-Lys-Arg (m = 1-10000),

à l'exception de la splénopentine ($R^1 = R^2 = R^3 = H$ ; $R^4 = OH$) et en présence d'une substitution en $N^{\alpha}$-Arg, d'une substitution en $N^{\epsilon}$-Lys ou d'une substitution en $N^{\alpha}$-Arg et en $N^{\epsilon}$-Lys,
avec un ou plusieurs adjuvants et/ou véhicules pharmacologiquement approprie's et/ou diluants physiologiquement acceptables.

**9.** Utilisation des splénopentines modifiées de formule I,

$R^1$-Arg-Lys($R^2$)-Glu-Val-Tyr($R^3$)$R^4$ (I),

dans laquelle:

$R^1$ représente l'hydrogène, un groupe alkyle en $C_{1-7}$, aryle en $C_{5-12}$, alkylaryle en $C_{6-20}$, aralkyle en $C_{6-20}$, alcanoyle en $C_{1-18}$, alcényle en $C_{1-7}$, alcynyle en $C_{1-7}$, polyhydroxyalcanoyle en $C_{6-12}$,
$-CO(CH_2)_n-COOH$ (n = 1-8),
$-CO(CHOH)_n-COOH$ (n = 1-12),
$-CO-(CH_2)_n-CO$-Arg-Lys-Glu-Val-Tyr (n = 1-8) ou
$-CO-(CHOH)_n-CO$-Arg-Lys-Glu-Val-Tyr (n = 1-12);

$R^2$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$, alcynyle en $C_{1-7}$, polyhydroxyalcanoyle en $C_{6-12}$,
$-CO(CH_2)_n-COOH$ (n = 1-8),
$-CO(CHOH)_n-COOH$ (n = 1-12),
$-CO(CH_2)_n-CO-(N^{\epsilon}$-Lys)-splénopentine (n = 1-8) ou
$-CO(CHOH)_n-CO-(N^{\epsilon}$-Lys)-splénopentine (n = 1-12);

$R^3$ représente l'hydrogène, un groupe alkyle en $C_{1-7}$, aryle en $C_{5-12}$, alkylaryle en $C_{6-20}$, alcanoyle en $C_{1-18}$, alcényle en $C_{1-7}$ ou alcynyle en $C_{1-7}$;

et

$R^4$ représente OH, $NH_2$, $NHR^5$, $NR^5_2$, ou $OR^5$, où $R^5$ représente:
un groupe alkyle en $C_{1-18}$, alcényle en $C_{1-7}$, alcynyle en $C_{1-7}$, aralkyle en $C_{6-20}$, alkylaryle en $C_{6-20}$,
$-(CHOH)_n$-polyhydroxyalkyle (n = 1-8),
$-(CH_2)_m$-NH-Tyr-Val-Glu-Lys-Arg (m = 1-18),
$-(CH_2CH_2-O)_m$-Tyr-Val-Glu-Lys-Arg (m = 1-10000) ou
$-(CH_2CH_2-O)_m$-NH-Tyr-Val-Glu-Lys-Arg (m = 1-10000),

a l'exception de la splénopentine ($R^1$ = $R^2$ = $R^3$ = H ; $R^4$ = OH) et en présence d'une substitution en $N^{\alpha}$-Arg, d'une substitution en $N^{\epsilon}$-Lys ou d'une substitution en $N^{\alpha}$-Arg et en $N^{\epsilon}$-Lys,

pour la préparation de médicaments pour la normalisation du système immunitaire, en particulier pour le traitement des infections virales, pour le traitement après des mesures chimiothérapeutiques, pour stimuler la croissance et la maturation des cellules médullaires, pour la thérapie des infections à HIV, pour empêcher des effets immunosuppresseurs dans les intoxications chroniques comme la consommation chronique d'alcool, et pour la thérapie des réactions auto-immunes.

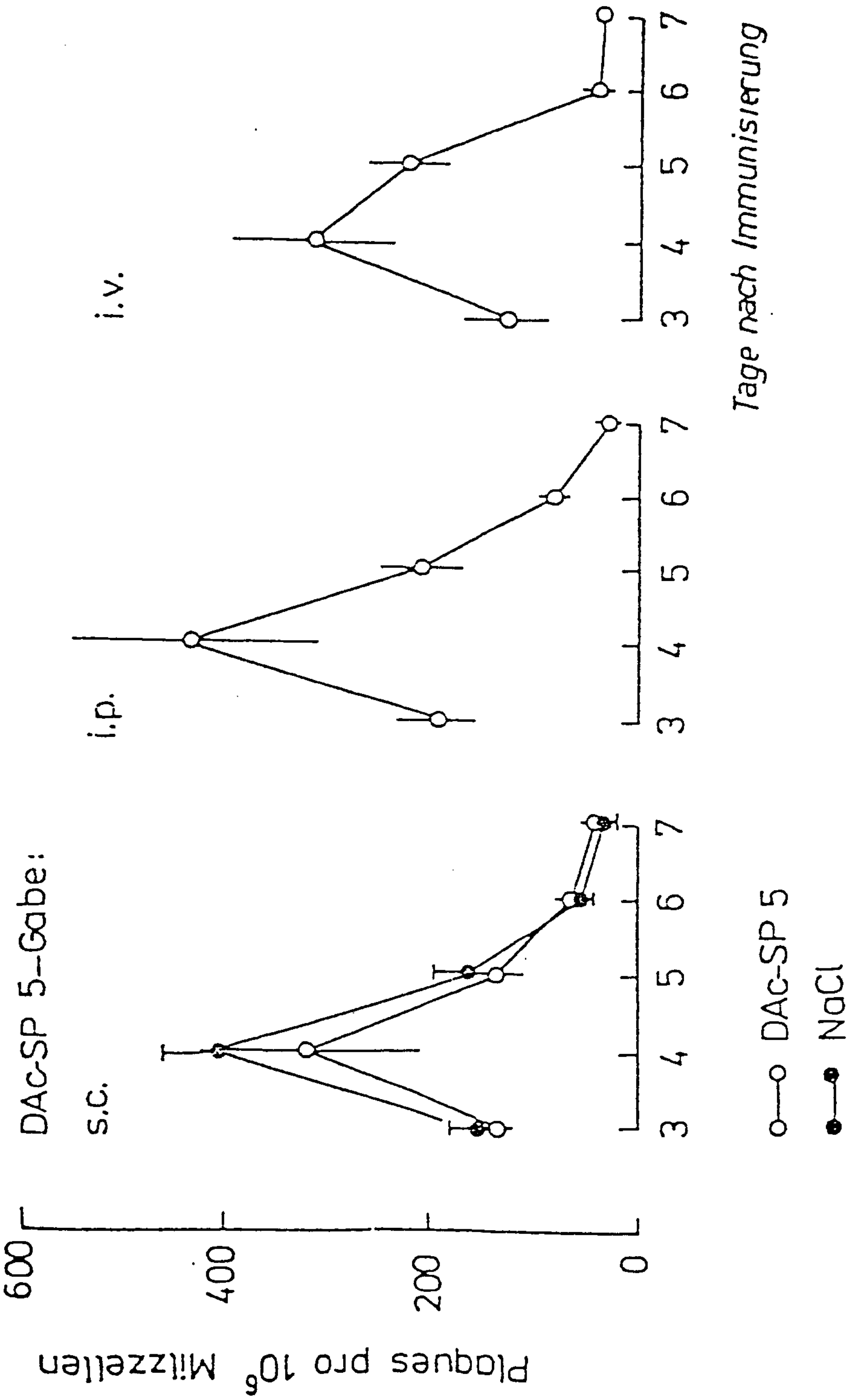
Plaques pro 10^6 Milzzellen
600
400
200
0
DAc-SP 5-Gabe:
s.c.
i.p.
i.v.
3 4 5 6 7
3 4 5 6 7
3 4 5 6 7
Tage nach Immunisierung
DAc-SP 5
NaCl

Fig.1

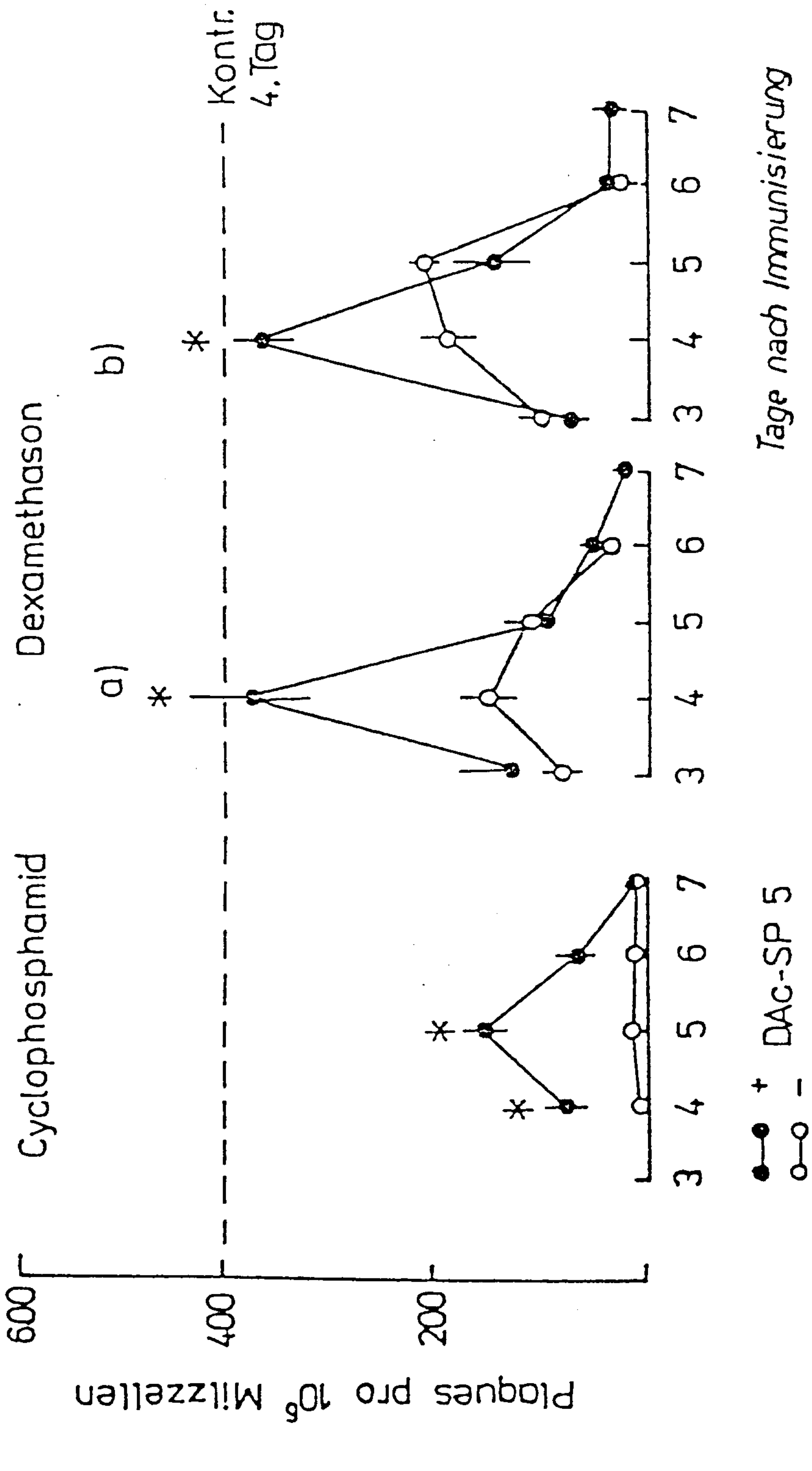
Cyclophosphamid
Dexamethason
a)
b)
Kontr.
4. Tag
Plaques pro 10⁶ Milzzellen
600
400
200
3 4 5 6 7
3 4 5 6 7
3 4 5 6 7
Tage nach Immunisierung
DAc-SP 5
+
–

Fig. 2

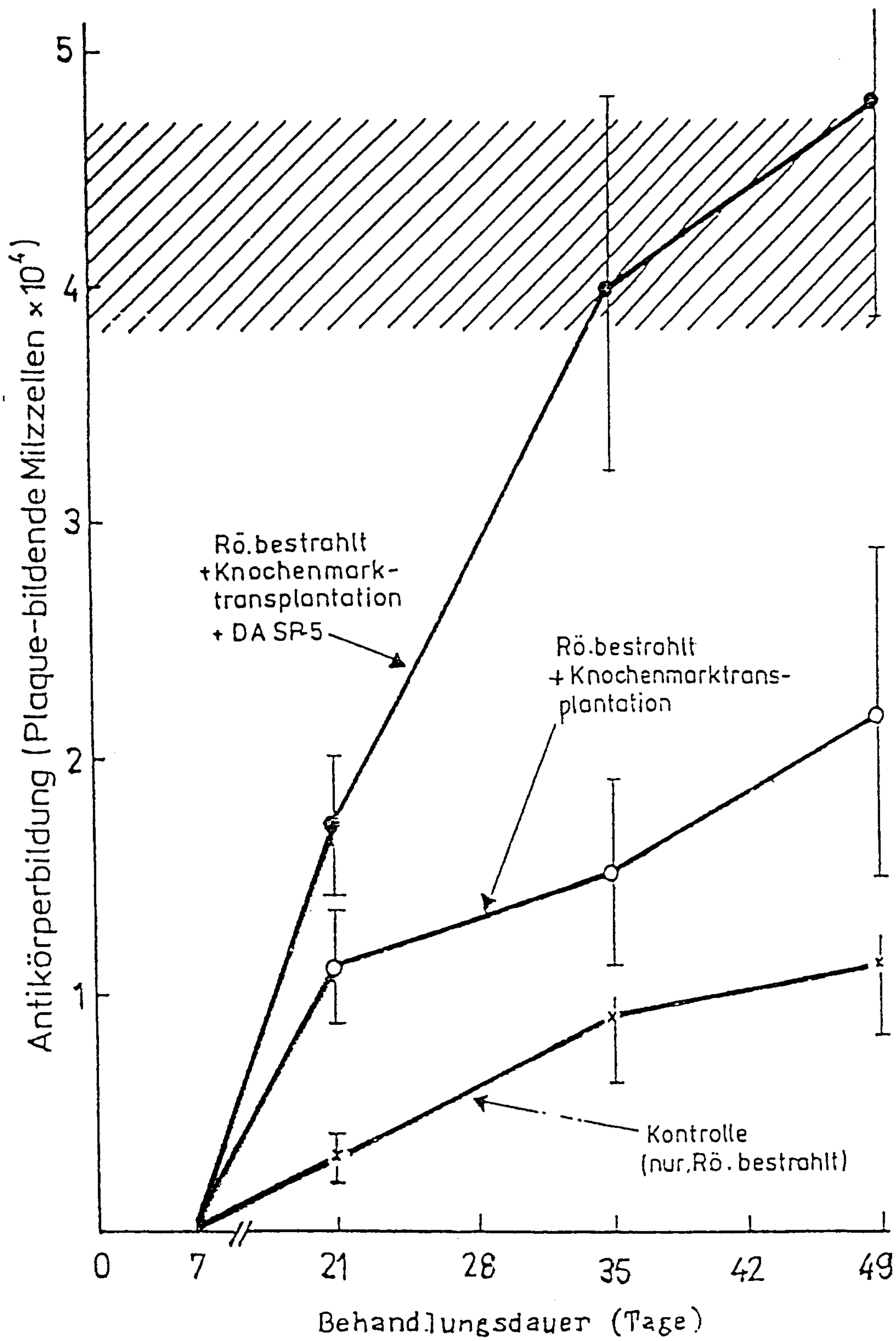
Antikörperbildung (Plaque-bildende Milzzellen ×10⁴)
5
4
3
2
1
Rö.bestrahlt
+Knochenmark-
transplantation
+ DA SP-5
Rö.bestrahlt
+Knochenmarktrans-
plantation
Kontrolle
(nur.Rö.bestrahlt)
0
7
21
28
35
42
49
Behandlungsdauer (Tage)

Fig.3

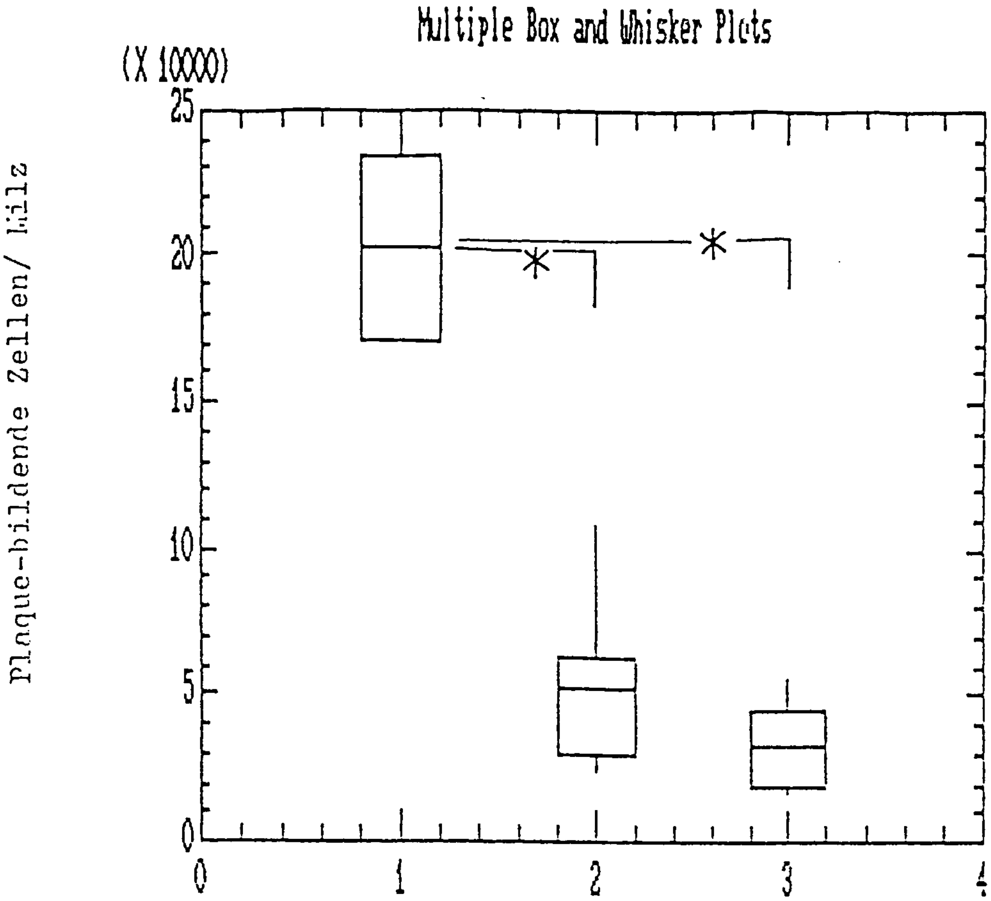
Multiple Box and Whisker Plots
(X 10000)
25
20
15
10
5
0
0
1
2
3
4
Plaque-bildende Zellen/ Milz

Fig. 4

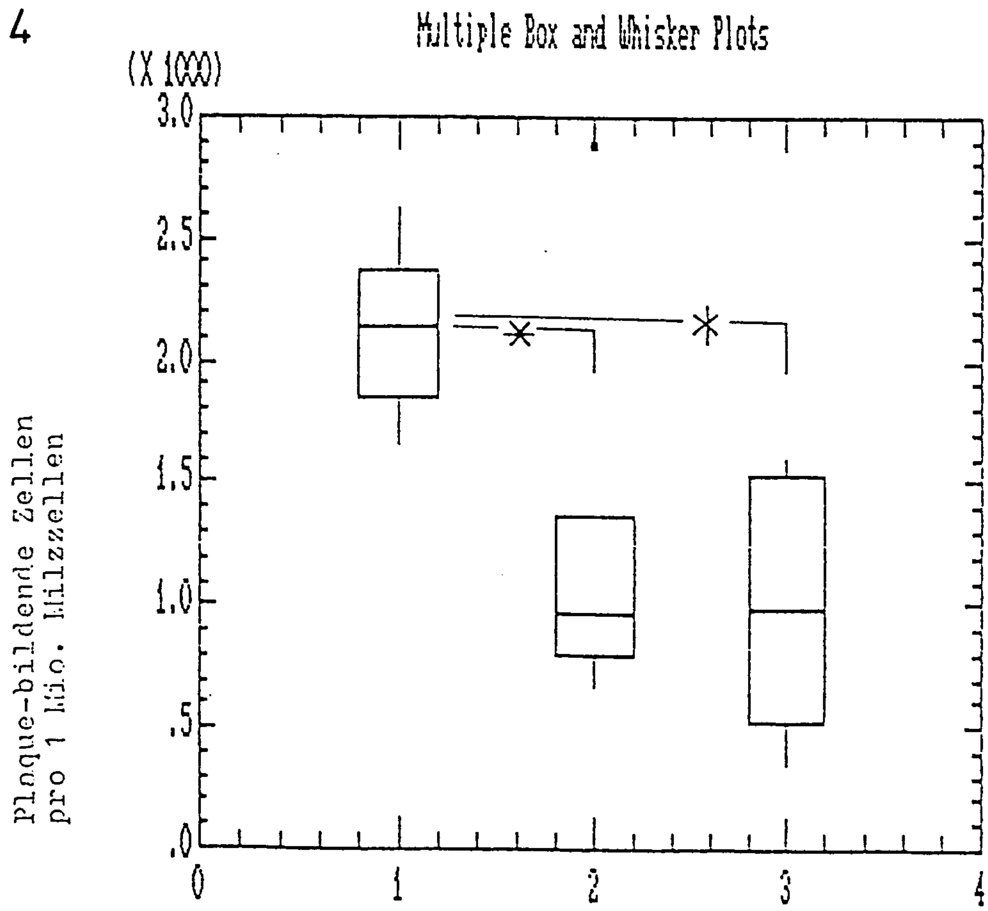
Multiple Box and Whisker Plots
(X 1000)
3.0
2.5
2.0
1.5
1.0
.5
.0
0
1
2
3
4
Plaque-bildende Zellen
pro 1 Mio. Milzzellen

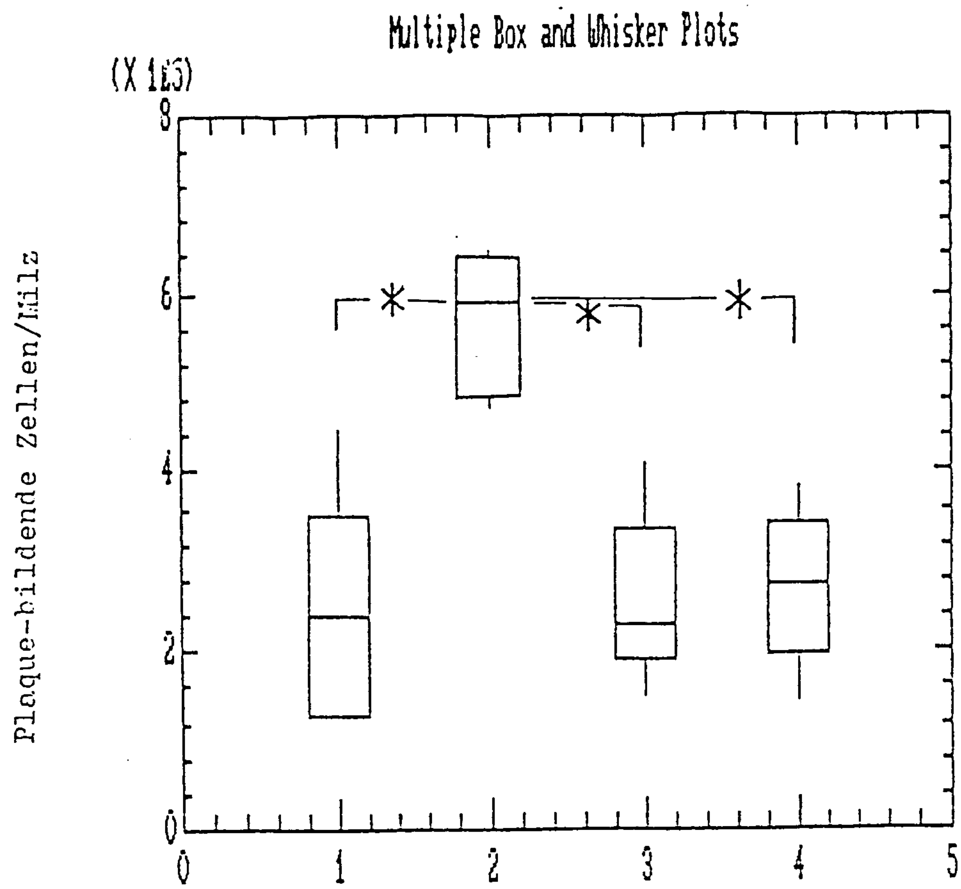

Multiple Box and Whisker Plots
(X 1E3)
Plaque-bildende Zellen/Milz
0
2
4
6
8
0
1
2
3
4
5


Fig.5

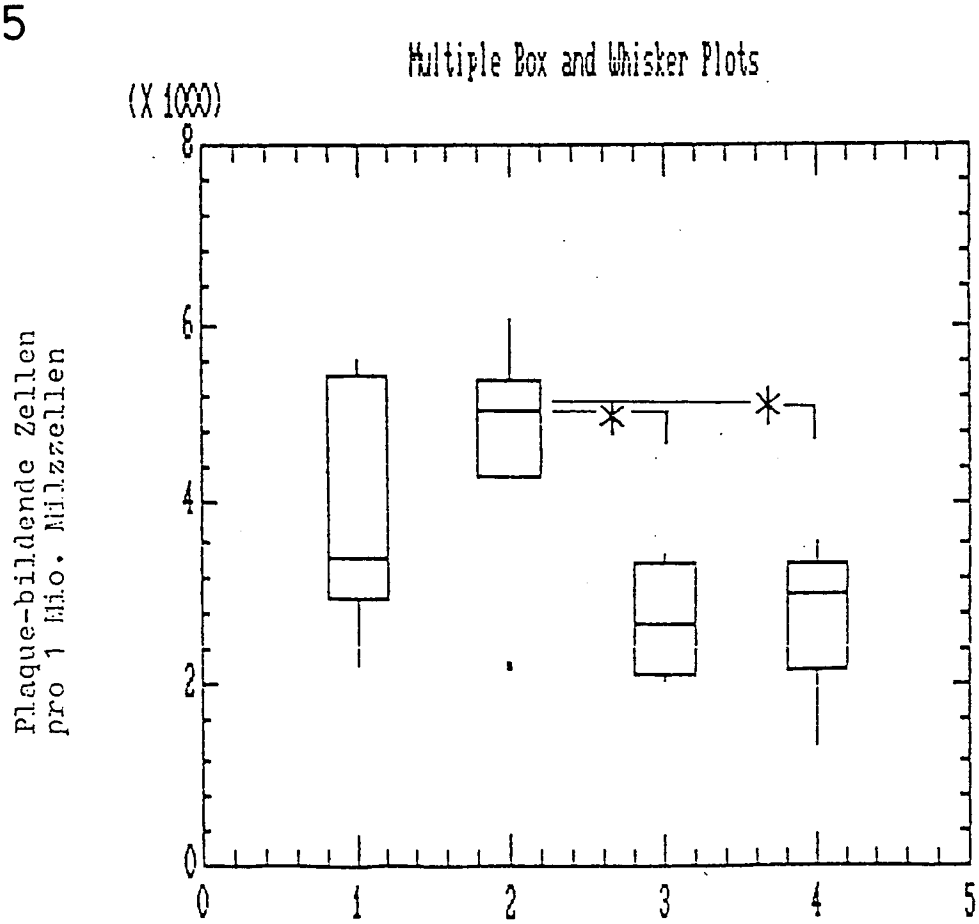

Multiple Box and Whisker Plots
(X 1000)
Plaque-bildende Zellen
pro 1 Mio. Milzzellen
0
2
4
6
8
0
1
2
3
4
5